(19) Europäisches Patentamt
European Patent Office
Office européen des brevets

(11) EP 3 084 066 B1

(12) # EUROPÄISCHE PATENTSCHRIFT

(45) Veröffentlichungstag und Bekanntmachung des Hinweises auf die Patenterteilung:
**25.10.2017 Patentblatt 2017/43**

(21) Anmeldenummer: **14824449.4**

(22) Anmeldetag: **18.12.2014**

(51) Int Cl.:
***D06M 13/513*** (2006.01) ***D06M 16/00*** (2006.01)
***C12Q 1/06*** (2006.01) ***C12Q 1/22*** (2006.01)

(86) Internationale Anmeldenummer:
**PCT/EP2014/078383**

(87) Internationale Veröffentlichungsnummer:
**WO 2015/091740 (25.06.2015 Gazette 2015/25)**

(54) **VERFAHREN ZUR VERMINDERUNG DER ADHÄSION VON MIKROORGANISMEN AN TEXTILIEN**
METHOD FOR REDUCING ADHESION OF MICROORGANISMS TO FABRICS
PROCÉDÉ DESTINÉ À RÉDUIRE L'ADHÉRENCE DE MICRO-ORGANISMES SUR DES TEXTILES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.12.2013 EP 13198710**

(43) Veröffentlichungstag der Anmeldung:
**26.10.2016 Patentblatt 2016/43**

(73) Patentinhaber:
- **Sanitized AG**
  **3400 Burgdorf (CH)**
- **Eidgenössische Materialprüfungs- und Forschungsanstalt EMPA**
  **9014 St. Gallen (CH)**

(72) Erfinder:
- **KATZENMEIER, Heinz**
  **CH-3432 Lützelflüh Goldbach (CH)**
- **STUTTE, Peter**
  **CH-3400 Burgdorf (CH)**
- **SCHMIDT-EMRICH, Sabrina**
  **CH-9008 St. Gallen (CH)**
- **THÖNY-MEYER, Linda**
  **CH-9053 Teufen (CH)**
- **ZULIAN, Qun Ren**
  **CH-9014 St. Gallen (CH)**

(74) Vertreter: **Jacobi, Markus Alexander**
**Isenbruck Bösl Hörschler LLP**
**Eastsite One**
**Seckenheimer Landstrasse 4**
**68163 Mannheim (DE)**

(56) Entgegenhaltungen:
**US-A- 3 860 709** **US-A1- 2005 227 092**
**US-A1- 2010 093 666**

- **DATABASE WPI Week 199714 Thomson Scientific, London, GB; AN 1997-148764 XP002740506, & JP H09 24089 A (TOYOBO KK) 28. Januar 1997 (1997-01-28)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 084 066 B1

## Beschreibung

Die vorliegende Erfindung betrifft ein Verfahren zur Ausrüstung von Fasern und/oder Textilien, durch das die Adhäsion von Mikroorganismen, insbesondere von Bakterien und/oder Hefen, an Fasern und/oder Textilien vermindert werden soll. Das Verfahren umfasst den Schritt des Aufbringens einer Zusammensetzung ZS, enthaltend ausgewählte hydrophile Silan-Derivate, auf die Fasern und/oder Textilien. Es wird zudem ein Verfahren zur quantitativen Bestimmung der Adhäsion von Mikroorganismen an Fasern und/oder Textilien beschrieben.

Die meisten Textilien enthalten mikrobiologisch abbaubares Material. Sie sind häufig entweder vollständig oder teilweise aus mikrobiologisch abbaubaren Fasern hergestellt, beispielsweise aus Baumwolle, Cellulose, z. B. Viskose, Lyocell (z.B. Tencel®), Hanf, Flachs, Leinen, Seide oder Wolle. Auch Textilien aus synthetischen Fasern, z.B. aus Polyester, Polyacrylnitril, Polyamid oder Polypropylen, werden regelmäßig von Bakterien besiedelt. Die Besiedelung von Textilien aus synthetischen Fasern erfolgt insbesondere dann, wenn diese Ausrüstungsmittel, wie z.B. Weichmacher, Hydrophobierungsmittel, Antistatika und/oder Binder, enthalten oder wenn sie während des Gebrauchs mikrobiologisch abbaubares Material aufnehmen, z.B. organische Substanzen aus dem Schweiß oder der Umwelt.

Insbesondere bei synthetischen Textilien, beispielsweise bei Polyestertextilien, ist zu beobachten, dass Bakterien an dem Textil bzw. der Faser haften und einen so genannten Biofilm ausbilden. Dieser Biofilm und die darin enthaltenden Bakterien können oftmals beim Waschen, insbesondere bei den für Polyestertextilien empfohlenen niedrigen Waschtemperaturen, nicht vollständig entfernt werden. Somit verbleiben Bakterien auf dem Textil, welche beim erneuten Tagen aktiv werden können und dann zu einer immer rascheren Bildung von unangenehmen Gerüchen führen. Diese Problematik verkürzt die Tragedauer von Polyestertextilien im Laufe der Zeit immer stärker und führt oftmals dazu, dass der unangenehme Geruch solcher Textilien nach einigen Tragezyklen trotz Waschens nicht mehr komplett entfernt werden kann. Dieses Phänomen ist in Fachkreisen wohl bekannt und wird oft als "alter Schweißgeruch" bezeichnet.

Unter einem Biofilm versteht man in der Regel einen Verbund von mikrobiellen Zellen, die mit einer Substratoberfläche verbunden sind, und die in einer extrazellulären polymeren Matrix (z.B. aus Exo-Polysacchariden), welche normalerweise durch die mikrobiellen Zellen selbst gebildet wird, eingebettet sind.

Die mikrobielle Besiedelung von Substraten und die Bildung eines Biofilms umfasst normalerweise die Adhäsion von Mikroorganismen, insbesondere Bakterien, z.B. das weit verbreitete *Pseudomonas sp.,* als einen ersten entscheidenden Schritt. Die Adhäsion der Bakterien oder der Proteinstrukturen der Bakterienhülle an verschiedenen Oberflächen erfolgt meist über unspezifischen hydrophobe Wechselwirkungen (z.B. Van der Waals Wechselwirkungen).

Die angelagerten Bakterien bilden durch die Abgabe von polymeren Substanzen, insbesondere Exo-Polysacchariden, mit der Zeit einen vollständig ausgebildeten Biofilm (auch als Bioschleim oder Schleimschicht bezeichnet). Dieser Biofilm ist gegenüber dem Einsatz von antimikrobiellen Wirkstoffen häufig sehr resistent, da die Schleimschicht in der Regel wenig durchlässig ist und die Mikroorganismen in der Schleimschicht eingebettet sind. Die Vermeidung einer initialen Adhäsion von Bakterien an Oberflächen stellt somit eine bevorzugte Herangehensweise zur Bekämpfung von Bakterien und der Verhinderung der Biofilm-Bildung dar.

Ein Vorteil einer Anti-Adhäsionsbeschichtung (oftmals auch als Anti-Fouling-Beschichtung bezeichnet) gegenüber der Ausrüstung mit antimikrobiellen Wirkstoffen, die direkt Bakterien abtöten oder deren Wachstum hemmen, besteht zudem darin, dass umweltfreundliche, nicht toxische Verbindungen, z. B. Polymere, eingesetzt werden können. Auf den Einsatz biologisch wirksamer und oftmals für den Menschen problematischer Wirkstoffe kann somit weitgehend verzichtet werden.

Anti-Fouling-Beschichtungen zur Verminderung der Adhäsion von Mikroorganismen an festen Substraten sind seit langer Zeit bekannt, insbesondere im medizinischen Bereich oder als Schutzanstrich für Schiffe. Im Stand der Technik sind beispielsweise Anti-Fouling-Beschichtungen aus Polyethylenglykol(PEG)-basierten Polymeren beschrieben, beispielsweise in M. Charnley et al. (Reactive & Functional Polymers 71 (2011), 329-334), Kingshott et al. (Langmuir 2003, 19, 6912-6921) oder WO 2008/089032.

Das Dokument WO 2003/024897 beschreibt ein Verfahren zur Beschichtung von Oberflächen, insbesondere hydrophoben Oberflächen, mit speziell gestalteten Thioethern und amphiphilen Thioethern.

Das Dokument WO 2008/049108 beschreibt multifunktionale Biocoatings und Verfahren zu deren Anwendung. Bei dem Oberflächen-modifizierenden Agens (SMA) kann es sich beispielsweise um Dopamin oder Dopamin-Derivate handeln. WO 2008/089032 ist gerichtet auf eine Antifouling-Beschichtung mit einem modifizierten Polyethylenglykol (PEG)-Polymer, z.B. ein Dopamin-modifiziertes PEG-Polymer wie PEG-$DOHA_4$.

Die Publikation Tsibouklis et al. (Contact Angle, Wettability and Adhesion, Vol. 4, 2006, 461-469) beschreibt die Bildung von Biofilmen auf Substraten und vergleicht Anti-Fouling-Beschichtungen aus Poly(fluoroalkyl(meth)acrylaten) und Poly(meth)acrylaten.

Das Dokument US 2010/0112364 beschreibt ein Verfahren zur Beschichtung von verschiedenen Substraten, z.B. Metallen, Textilien, Kunststoffen, wobei die Oberfläche des Substrats oxidiert wird, danach ein ungesättigtes Monomer aufgetragen und polymerisiert wird. Die Beschichtung soll die Adhäsion von biologischem Material vermindern.

Die im Stand der Technik beschriebenen Beschichtungsverfahren sind entweder technisch aufwendig und/oder

kostenintensiv durchzuführen und/oder liefern eine nicht ausreichend wirksame und stabile anti-adhäsive Beschichtung. Insbesondere sind die im Stand der Technik beschriebenen Verfahren nicht dazu geeignet, eine stabile (insbesondere waschstabile) Ausrüstung von Fasern und/oder Textilien bereitzustellen, die einfach und kostengünstig mittels gängiger textiler Applikationsmethoden aufgebracht werden kann.

Weiterhin ist es für die Entwicklung eines solchen Beschichtungsverfahrens entscheidend die anti-adhäsive Wirksamkeit der Faser- bzw. Textilbeschichtung verlässlich und schnell, insbesondere in einem Hochdurchsatzverfahren, bestimmen zu können.

Das Dokument US-B 3,860,709 beschreibt ein Verfahren zur Verminderung des Bakterien- und Pilzwachstums unter Verwendung von speziellen antimikrobiellen Organosiloxan-Aminen, insbesondere die Verbindung $[(CH_3)_3SiO]_3Si(CH_2)_3NHCH_2CH_2NH_2$, wobei die Bakterien oder Pilze mit den Organosiloxan-Aminen in Kontakt gebracht werden.

Das Dokument US-A 2010/0093666 offenbart wasserstabile Zusammensetzungen enthaltend eine Organosilan-Verbindung und eine stabilisierende Lösung enthaltend eine anorganische Säure, einen Glykolether und ein kationisches Tensid, wobei es sich bei dem kationischen Tensid bevorzugt um ein quarternäres Ammoniumsalz (QAS) handelt.

Das Dokument US-A 2005/0227092 beschreibt Beschichtungszusammensetzungen und Antifouling-Beschichtungen enthaltend ein spezielles Organosiloxan-Derivat. Die Beschichtungszusammensetzungen werden auf ein Substrat aufgetragen und ausgehärtet.

Im Stand der Technik wird eine Vielzahlt von Verfahren zur quantitativen Bestimmung der Biofilmbildung beschrieben, bzw. von Verfahren zur Bestimmung der Biomasse eines bestehenden Biofilms. Insbesondere im medizinischen Bereich, wie etwa bei Implantaten, ist die Bildung von Biofilmen, z.B. durch Mikroorganismen der Gattung *Staphylococcus,* kritisch, da Bakterien in einem Biofilm grundsätzlich andere Eigenschaften aufweisen, als suspendierte Bakterien, z.B. eine höhere Resistenz gegenüber Antibiotika.

Das Dokument JP-H0924089 beschreibt ein Verfahren zur Bestimmung der antimikrobiellen Funktion von Textilien, wobei eine Bakteriensuspension auf eine sterilisierte Textilprobe aufgetragen und getrocknet wird.

Die Publikation Srdjan S. et al (APMIS, 115: 891-899, 9, 2007) beschreibt die einzelnen Schritte eines Verfahren zur quantitativen Bestimmung der Biofilm-Bildung von *Staphylococci* in verschiedenen Mikrotiterplatten, z.B. in Mikrotiterplatten, die mit Gewebekulturen behandelt wurden. Es wird ein Verfahren beschrieben, bei dem ein Biofilm an den Wänden und Böden der Mikrotiterplatte gebildet wird und direkt in der Mikrotiterplatte mittels eines geeigneten Farbstoffes, z.B. mit Kristallviolett, angefärbt wird. Die Menge des angelagerten Farbstoffs wird durch ein geeignetes Verfahren (z.B. mittels Mikrotiterplatten-Reader) ausgewertet.

Die Publikation Peeters E. et al (Journal of Microbiological Methods 72 (2008) 157-165) vergleicht verschiedene Verfahren zur quantitativen Bestimmung von Bio filmen, die in einer Mikrotiterplatte gewachsen sind. Es wird unter anderem beschrieben, dass Biofilme von *P.aesuginaosa* und *S. aureus* mit verschiedenen Farbstoffen, z.B. Kristallviolett, Syto9, direkt in den Mikrotiterplatten angefärbt und mittels geeigneter spektroskopischer Verfahren ausgewertet werden können.

Die Publikation Kingshott et al. (Langmuir 2003, 19, 6912-6921) beschreibt ein Verfahren zur Bestimmung der Adhäsion von *Pseudomonas sp.* an modifizierten PET-Kunststoffoberflächen, wobei die Anzahl der angelagerten Bakterien durch indirekte Konduktometrie anhand des von den angelagerten Bakterien produzierten Kohlendioxid bestimmt wird. Die Publikation Ciag et al (Langmuir 2012, 28, 1399-1407) beschreibt ein Bakterien-Adhäsions-Assay, wobei die auf einer PEG-modifizierten Oberfläche angelagerten Bakterien angefärbt und mittel Konfokal-mikroskopie (confocal laser scanning microscopy, CLSM) untersucht werden.

Die im Stand der Technik beschriebenen Bestimmungsverfahren sind nicht für Textilen geeignet und oft auf spezielle Mikrotiterplatten-Systeme beschränkt. Oftmals betreffen die Bestimmungsverfahren des Stand der Technik Verfahren, bei denen der Biofilm oder die Anzahl der angelagerten Bakterien direkt auf dem Substrat bestimmt wird, was oftmals einen hohen apparativen und zeitlichen Aufwand benötigt.

Eine Aufgabe der Erfindung besteht somit darin ein neues Ausrüstungs-, insbesondere Beschichtungsverfahren, insbesondere für Fasern und/oder Textilien, unter Verwendung von nicht-toxischen, umweltverträglichen Beschichtungsagenzien, bereitzustellen. Die Beschichtung soll wirksam die Adhäsion von Mikroorganismen reduzieren und eine hohe Stabilität, insbesondere Waschbeständigkeit auf Fasern und/oder Textilien, aufweisen. Es ist zudem wünschenswert, dass die Ausrüstung, insbesondere Beschichtung der Fasern und/oder Textilien mittels üblicher textiler Ausrüstungsverfahren einfach und kostengünstig durchgeführt werden kann.

Es wird zudem ein Verfahren zur quantitativen Bestimmung der Adhäsion von Mikroorganismen an Fasern und/oder Textilienbeschrieben, mittels dessen die Wirksamkeit des Beschichtungs-verfahrens überprüfen und verlässlich quantitativ bestimmt werden kann. Das Bestimmungsverfahren sollte zuverlässig und einfach für alle Arten von Fasern und Textilien und für verschiedenen Mikroorganismen, insbesondere für Bakterien und Hefen, angewendet werden können. Es ist zudem wünschenswert, dass das Verfahren zur quantitativen Bestimmung der Adhäsion von Mikroorganismen an Fasern und/oder Textilien in Form eines Hochdurchsatz-Verfahrens (z.B. als High-Throughput-Verfahren) durchgeführt werden kann.

Die vorliegende Erfindung betrifft ein Verfahren zur Ausrüstung von Fasern und/oder Textilien umfassend die

Schritte:

a) Bereitstellen einer Zusammensetzung ZS enthaltend mindestens ein hydrophiles Silan-Derivat S der allgemeinen Formel (I)

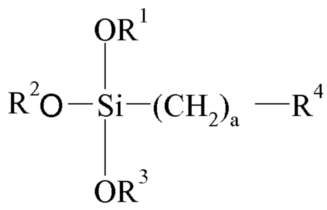

(I)

wobei

$R^1$, $R^2$ und $R^3$ unabhängig voneinander H oder $C_1$-$C_6$-Alkyl, bevorzugt Methyl oder Ethyl, sind;
a eine ganze Zahl von 1 bis 10, bevorzugt von 2 bis 6, besonders bevorzugt 3, ist;

$R^4$ ausgewählt ist aus:

i)

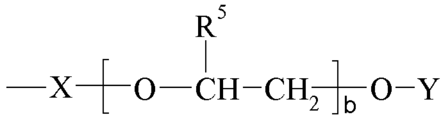

mit

X ist eine Bindung, -O-T-, -O-T-CH(OH)-, -O-T-CH(OH)-T'-, -O-C(=O)- oder -O-C(=O)-T-, wobei T und T' unabhängig voneinander ausgewählt sind aus $C_{1-12}$-Alkylen, bevorzugt aus $C_{1-6}$-Alkylen,
$R^5$ ist unabhängig voneinander H oder $C_{1-6}$-Alkyl, bevorzugt H oder Methyl;
Y ist H; $C_{1-12}$-Alkyl; $C_{1-12}$-Hydroxyalkyl, $C_{1-12}$-Haloalkyl, $C_{7-20}$-Arylalkyl, -C(=O)($C_{1-12}$-Alkyl); $-(CH_2)_a$-Si($OR^1$)($OR^2$)($OR^3$), -T-O-$(CH_2)_a$-Si($OR^1$)($OR^2$)($OR^3$); -T-CH(OH)-O-$(CH_2)_a$-Si($OR^1$)($OR^2$)($OR^3$), oder -T-CH(OH)-T'-O-$(CH_2)_a$-Si($OR^1$)($OR^2$)($OR^3$) mit $R^1$, $R^2$, $R^3$, T, T' und a wie oben definiert;
b ist eine Zahl von 0 bis 20, bevorzugt 2 bis 10;

oder (xv)

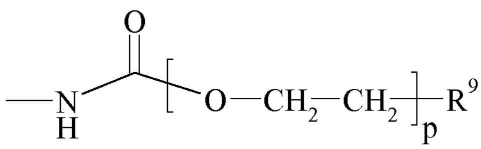

mit p = 1 bis 20, und $R^9$ ist ausgewählt aus -OH, $-OCH_3$, $-OCH_2CH_3$ und $-NH_2$;
und optional mindestens ein Lösungsmittel L;

b) Aufbringen der Zusammensetzung ZS auf die Fasern und/oder Textilien;

c) optional Waschen und/oder Trocknen der Fasern und/oder Textilien.

Mit Hilfe des erfindungsgemäßen Verfahrens ist es möglich Fasern und/oder Textilien mit einer anti-adhäsiven Beschichtung auszurüsten, wobei die Anlagerung/Adhäsion von Bakterien an der Oberfläche der Fasern und/oder Textilien und die Ausbildung eines Bakterienfilms (Biofilm) wirksam vermieden werden kann.

Es hat sich gezeigt, dass mit Hilfe des erfindungsgemäßen Verfahrens eine besonders stabile und waschfeste Ausrüstung möglich ist, welche auch nach mehrmaligem Waschen, z.B. 10 bis 100 Wäschen, und/oder mechanischer Beanspruchung, wirksam die Adhäsion von Bakterien verringert.

Es wurde gefunden, dass die Adhäsion von einer Vielzahl von häufig vorkommenden Bakterien, wie beispielsweise:

*Pseudomonas aeruginosa (P. aeruginosa), Escherichia coli (E. coli), Staphylococcus aureus (S. aureus), Staphylococcus epidermidis (S. epidermidis)* und *Candida albicans (C. albicans),*

verhindert oder deutlich verringert werden kann. Die eingesetzten hydrophilen Silan-Derivate zeichnen sich zudem durch ihre toxikologische Unbedenklichkeit und eine gute Umwelt-Verträglichkeit aus.

Besonders vorteilhaft ist, dass bei der Ausrüstung nach dem erfindungsgemäßen Verfahren auf den Einsatz von gängigen antimikrobiellen Wirkstoffen (z. B. Silbersalze, Thiohydroxamsäure-Derivate, Isothiazolinon-Derivate oder Benzimidazol-Derivate) verzichtet werden kann. Es wurde gezeigt, dass eine Beschichtung mit den verwendeten Silan-Derivaten selbst keine antimikrobielle Wirkung aufweist.

Unter einer antimikrobiellen Wirkung wird im Sinne der vorliegenden Erfindung verstanden, dass eine direkte Wechselwirkung mit Mikroorganismen, z.B. Bakterien, Hefen und/oder Viren, stattfindet, welche zu einer Abtötung oder einer verminderten Vermehrung der Mikroorganismen führt.

Insbesondere handelt es sich bei dem erfindungsgemäßen Verfahren zur Ausrüstung von Fasern und/oder Textilien um ein Verfahren zu anti-adhäsiven Ausrüstung, wobei die Adhäsion (Anlagerung) von Mikroorganismen an den Fasern und/oder Textilien verhindert oder vermindert werden soll.

Insbesondere handelt es sich bei den Mikroorganismen um Bakterien, Viren, Pilzen, Hefen und Algen, insbesondere um Bakterien und Hefen. Bevorzugt handelt es sich um Mikroorganismen, insbesondere um Bakterien und Hefen, die typischerweise die Haut des Menschen und von Tieren besiedeln.

Unter Faser (Faserrohstoff) versteht man im Sinne der Erfindung linienförmige Gebilde, welche die kleinste Einheit von Textilien bzw. Textilmaterialien bilden, wobei zwischen Spinnfasern (spinnbaren Fasern, Fasern mit begrenzter Länge) und Filamenten (gezogenen Endlosfasern) unterschieden werden kann. Fasern können beispielsweise mineralische oder pflanzliche Naturfasern, z.B. Baumwolle, Wolle, Seide, oder künstliche Fasern (Chemiefasern) hergestellt aus synthetischen Polymeren, z.B. Polyester, Polyamid, oder aus Polymeren natürlichen Ursprungs, z.B. Cellulose-Regeneratfasern, sein.

Unter Textilien versteht man im Sinne der Erfindung Gefüge (Gebilde) aus Fasern, welche typischerweise aus einem textilen Verarbeitungsprozess hervorgehen, wobei der Verarbeitungsprozess insbesondere Spinnen, Weben, Wirken und daraus abgewandelte und darauf aufbauende Prozesse umfasst. Unter Textilien versteht man textile Halb- und Fertigwaren, wie z.B. ungeformte Gebilde, z.B. Flocken; linienförmige Gebilde, z:B. Fäden, Garne, Zwirne, Seile, und flächenförmige Gebilde, z.B. Gewebe, Vliese, Filzstoffe.

Unter einem Silan-Derivat im Sinne der vorliegenden Erfindung versteht man eine Silicium-organische Verbindung umfassend Si-C und Si-O Bindungen. Insbesondere handelt es sich im Sinne der Erfindung um ein hydrophiles Silan-Derivat, wobei es sich bevorzugt bei dem Rest $R^4$ gemäß Formel I um einen hydrophilen Rest handelt.

Im Sinne der vorliegenden Erfindung sind die genannten Reste wie folgt definiert:

Alkyl bezeichnet einen univalenten Rest bestehend aus einer linearen, verzweigten oder cyclischen Kohlenwasserstoffgruppe, bevorzugt aus einer linearen oder verzweigten Kohlenwasserstoffkette, insbesondere umfassend 1 bis 12 Kohlenstoffatome. Beispielsweise kann es sich bei dem Alkylrest um Methyl, Ethyl, n-Propyl oder iso-Propyl handeln.

Arylalkyl bezeichnet einen univalenten Rest abgeleitet von einem linearen oder verzweigten Alkylrest, insbesondere umfassend 1 bis 14 Kohlenstoffatome, durch den Austausch eines oder mehrerer Wasserstoffatome durch eine Arylgruppe, wobei die Arylgruppe eine substituierte oder unsubstituierte aromatische Kohlenwasserstoff-Gruppe, insbesondere umfassend 6 Kohlenstoffatome, ist. Beispielsweise kann es sich bei der aromatische Kohlenwasserstoff-Gruppe um Phenyl handeln; beispielsweise kann es sich bei dem Arylalkyl-Rest um einen Benzyl-Rest handeln.

Haloalkyl bezeichnet einen univalenten Rest abgeleitet von einem von einem linearen oder verzweigten Alkylrest, insbesondere umfassend 1 bis 12 Kohlenstoffatome durch den Austausch eines oder mehrerer Wasserstoffatome durch ein Halogen (-F, -Cl, -Br, -I, insbesondere Cl). Halogen bezeichnet einen Substituenten ausgewählt aus Fluorid, Chlorid, Bromid oder Iodid, insbesondere Chlorid.

Hydroxyalkyl bezeichnet einen univalenten Rest abgeleitet von einem von einem linearen oder verzweigten Alkylrest, insbesondere umfassend 1 bis 12 Kohlenstoffatome durch den Austausch eines oder mehrerer Wasserstoffatome durch eine Hydroxygruppe (-OH). Alkylen, Arylalkylen, Haloalkylen, Hydroxyalkylen bezeichnen die entsprechenden divalenten Reste.

Die Reste $R^1$, $R^2$, $R^3$ in Formel (I) sind unabhängig voneinander ausgewählt aus H oder $C_1$-$C_6$-Alkyl, bevorzugt aus $C_1$-$C_3$-Alkyl, besonders bevorzugt aus Methyl oder Ethyl. Insbesondere bevorzugt ist $R^1 = R^2 = R^3$, bevorzugt $R^1 = R^2 = R^3$ = Methyl oder Ethyl.

Eine bevorzugte Ausführungsform betrifft ein Verfahren wie oben beschrieben, wobei $R^1$, $R^2$ und $R^3$ unabhängig voneinander Methyl oder Ethyl sind und a = 3 ist.

Die Reste $R^5$ und $R^{10}$ sind unabhängig voneinander H oder $C_{1-6}$-Alkyl. Bei den Resten $R^5$ und/oder $R^{10}$ handelt es sich bevorzugt um H oder $C_{1-3}$-Alkyl, insbesondere bevorzugt um H, Methyl oder Ethyl, besonders bevorzugt um H oder Methyl. Die Orientierung der Alkylenoxygruppen $-(O-CH(R^5)-CH_2)_b$ und $-(O-CH(R^{10})-CH_2)_p$ gemäß Formel (I) kann beliebig sein. Der Fachmann weiß, dass beim Einbau der entsprechenden Alkylenoxide, z.B. Propylenoxid und/oder Butylenoxid, beiden Verknüpfungsrichtungen möglich sind. Zudem weiß der Fachmann, dass es sich bei der Anzahl der Alkylenoxygruppen, d.h. bei den Indizes b und p, um Mittelwerte handelt.

Bei den Index b handelt es sich um eine Zahl von 0 bis 20, bevorzugt 2 bis 15, besonders bevorzugt von 2 bis 10, beispielsweise bevorzugt von 9 bis 12, beispielsweise bevorzugt von 6 bis 9, beispielsweise bevorzugt von 4 bis 6.

Bei den Index p handelt es sich um eine Zahl von 1 bis 20, bevorzugt 1 bis 15; besonders bevorzugt von 2 bis 10, beispielsweise bevorzugt von 9 bis 12, beispielsweise bevorzugt von 6 bis 9, beispielsweise bevorzugt von 4 bis 6.

Bei dem Rest $R^4$ handelt es sich insbesondere um einen hydrophilen Rest, insbesondere um einen Rest umfassend Alkoxygruppen, Hydroxygruppen, Aminogruppen und/oder ionische Gruppen, insbesondere anionische Gruppen.

In einer bevorzugten Ausführungsform wird ein hydrophiles Silan-Derivat S gemäß der oben beschriebenen Formel I eingesetzt, wobei $R^4$ die Struktur i)

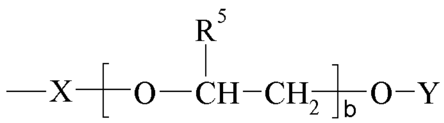

aufweist
mit Y, $R^5$ und b wie oben definiert und

X ist eine Bindung, -O-T-, -O-T-CH(OH)-, -O-T-CH(OH)-T'-, -O-C(=O)-oder -O-C(=O)-T-, wobei T und T' unabhängig voneinander ausgewählt sind aus $C_{1-12}$-Alkylen, bevorzugt aus $C_{1-6}$-Alkylen, besonders bevorzugt aus $C_{1-3}$-Alkylen, insbesondere bevorzugt sind T und/oder T' $-CH_2-$.

In einer bevorzugten Ausführungsform ist der divalente Rest X ausgewählt aus einer Bindung, $-O-(CH_2)_n-$, $-O-(CH_2)_n-CH(OH)-$, oder $-O-(CH_2)_{n'}-CH(OH)-(CH_2)_n-$, $-O-C(=O)-(CH_2)_n-$, wobei n und n' unabhängig voneinander eine ganze Zahl von 1 bis 6, bevorzugt n=1 und n'=1, sind. Besonders bevorzugt ist der divalente Rest -X- ausgewählt aus einer Bindung oder $-O-CH_2-CH(OH)-CH_2-$.

Bevorzugt ist die Orientierung des divalenten Restes -X- in der Gruppe i)

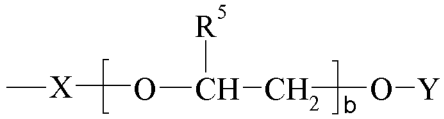

wie angegeben (Leserichtung von links nach rechts), wobei die linke freie Bindung an die Gruppe $-(CH_2)_a-$ von Formel (I) und die rechte freie Bindung an die Gruppe $-[O-CH(R^5)-CH_2]_b-$ gebunden ist.

Insbesondere weist die oben genannte Gruppe i) eine der folgenden Strukturen (i1) bis (i7) auf:

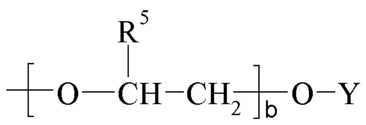

(i1),

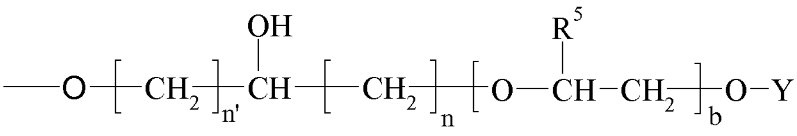

(i2),

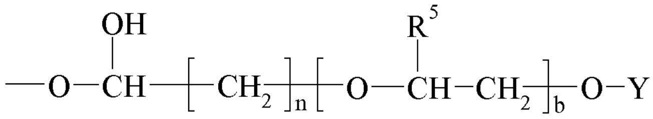

(i3),

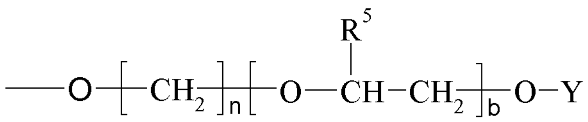

(i4),

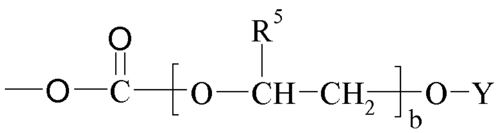

(i5),

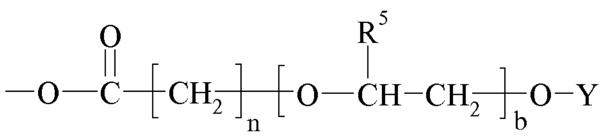

(i6),

-O-Y (i7),

wobei die Reste und Indizes die oben genannten Bedeutungen haben. Bevorzugt wird in dem erfindungsgemäßen Verfahren zur Ausrüstung von Fasern und/oder Textilien ein hydrophiles Silan-Derivat S eingesetzt, wobei die oben genannte Gruppe i) eine der Strukturen (i1) oder (i2) aufweist.

Insbesondere betrifft die Erfindung ein oben beschriebenes Verfahren, wobei $R^4$ eine Gruppe ist ausgewählt aus:

(x)

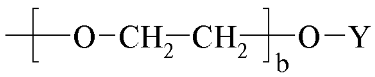

mit b und Y wie oben definiert; bevorzugt mit b = 2 bis 20 und Y ist ausgewählt aus H und $C_1$-$C_6$-Alkyl, bevorzugt aus H, Methyl und Ethyl;

(xi)

-O-Y

mit Y wie oben definiert; bevorzugt mit Y ist ausgewählt aus H, $C_{1\text{-}6}$-Alkyl und -C(=O)($C_{1\text{-}12}$-Alkyl), bevorzugt aus H, Methyl, Ethyl und Acetyl;

(xii)

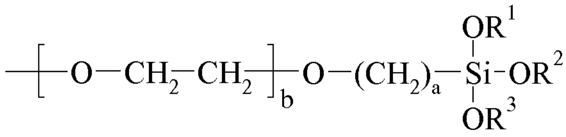

mit $R^1$, $R^2$, $R^3$, a und b wie oben definiert; bevorzugt mit a = 2 bis 6, bevorzugt 3, bevorzugt mit b = 2 bis 10; bevorzugt sind $R^1$, $R^2$ und $R^3$ unabhängig voneinander Methyl oder Ethyl;

(xiii)

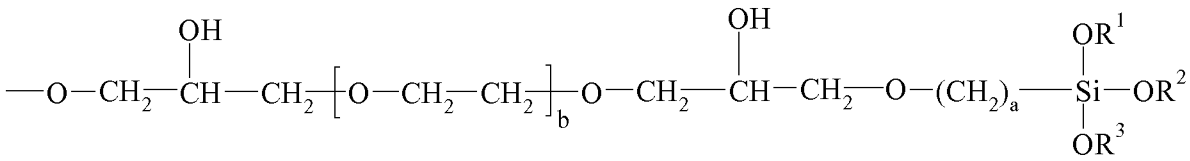

mit $R^1$, $R^2$, $R^3$, a und b wie oben definiert; bevorzugt mit a = 2 bis 6, bevorzugt 3, bevorzugt mit b = 2 bis 20, bevorzugt 5 bis 10, und $R^1$, $R^2$, $R^3$ sind bevorzugt unabhängig voneinander Methyl oder Ethyl;

Bevorzugt wird mindestens eine der folgenden Verbindungen der Formeln (S1a) bis (S3a) als hydrophiles Silan-Derivat S eingesetzt:

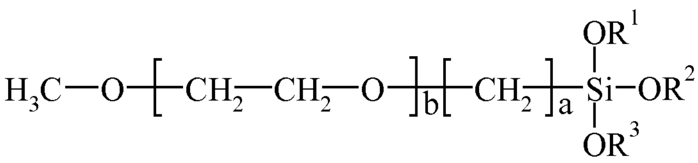

(S1a),

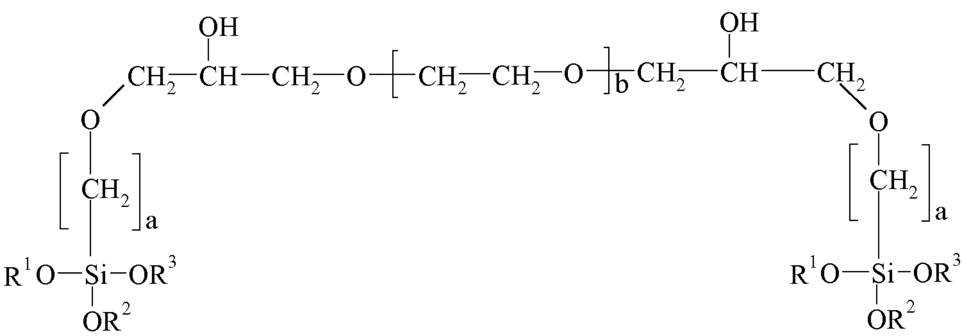

(S2a),

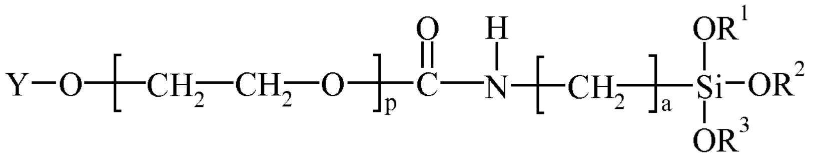

(S3a),

wobei die Reste und Indizes die oben genannten Bedeutungen haben. Insbesondere gelten für die Verbindungen der Formeln (S1a) bis (S3a) die oben genannten bevorzugten Ausführungsformen der Reste und Indizes.

In einer bevorzugten Ausführungsform wird mindestens eine Verbindungen der Formel (S3a) als hydrophiles Silan-Derivat S eingesetzt.

Bevorzugt wird mindestens eine der folgenden Verbindungen (S1), (S2), (S3), (S6), (S10) und (S11) als hydrophiles Silan-Derivat S eingesetzt:

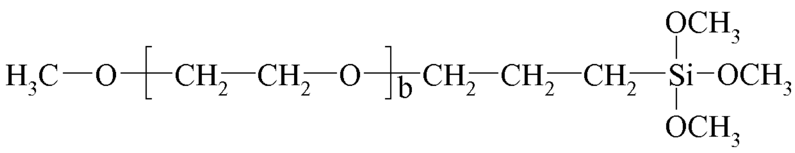

mit b = 0-20, bevorzugt b= 5-10

(S1)

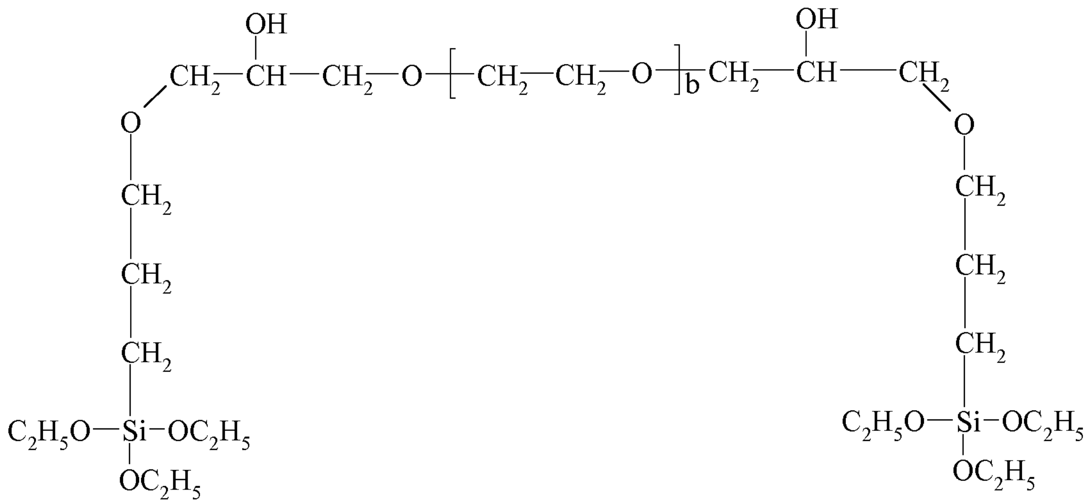

mit b = 0-20, bevorzugt b = 5-10

(S2)

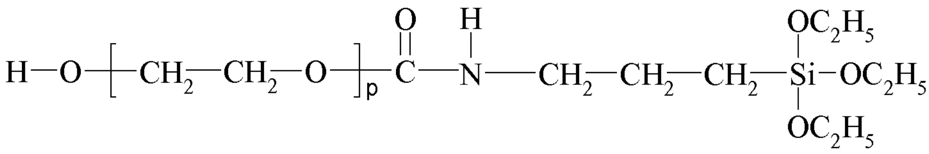

mit p = 1 bis 20, bevorzugt p = 4 - 6

(S3)

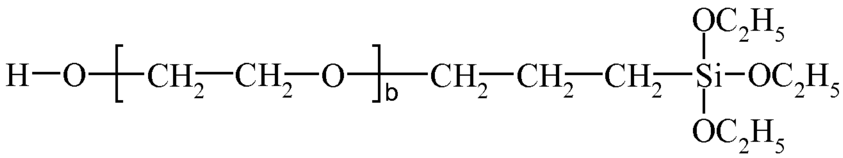

mit b = 1 bis 20, bevorzugt b = 6 - 9

(S6)

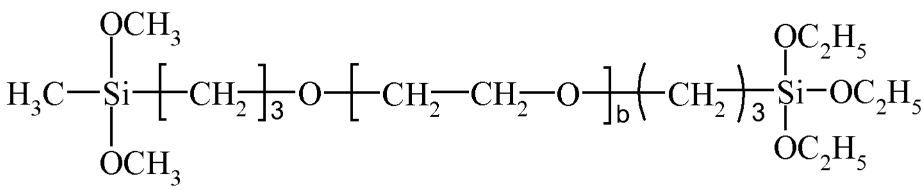

mit b = 1 bis 20, bevorzugt b = 6 - 9

(S10)

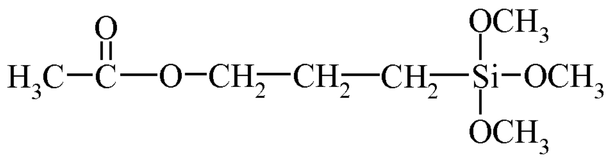

(S11).

Bevorzugt betrifft die Erfindung ein Verfahren zur Ausrüstung von Fasern und/oder Textilien, wobei die Zusammensetzung ZS mindestens ein hydrophiles Silan-Derivat S ausgewählt aus den Verbindungen S1 bis S3 enthält.

In einer besonders bevorzugten Ausführungsform betrifft die Erfindung ein Verfahren zur Ausrüstung von Fasern und/oder Textilien, wobei die Zusammensetzung ZS mindestens ein hydrophiles Silan-Derivat S3 enthält, insbesondere kann die Zusammensetzung ZS als hydrophiles Silan-Derivat ausschließlich S3 enthalten.

Das erfindungsgemäße Verfahren umfasst das Bereitstellen der Zusammensetzung ZS enthaltend ein oben beschriebenes hydrophiles Silan-Derivat S der Formel (I) und optional mindestens ein Lösungsmittel L. Bevorzugt besteht die Zusammensetzung ZS aus mindestens einem oben beschriebenen hydrophilen Silan-Derivat S der Formel (I) und mindestens einem Lösungsmittel L.

Bevorzugt wird in dem erfindungsgemäßen Verfahren eine Zusammensetzung ZS eingesetzt, welche 0,00001 bis 100 Gew.-%, bevorzugt 0,0001 bis 10 Gew.-%, besonders bevorzugt 0,001 bis 5 Gew.-%, insbesondere bevorzugt 0,01 bis 1 Gew.-%, weiterhin bevorzugt 0,001 bis 0,1 Gew.-% des mindestens einen hydrophilen Silan-Derivats S, bezogen auf die gesamte Zusammensetzung ZS, enthält.

Bevorzugt wird in dem erfindungsgemäßen Verfahren das mindestens eine hydrophile Silan-Derivat S in einer Konzentration (Applikationskonzentration) von 0,00001 bis 5 Gew.-%, bevorzugt von 0,0001 bis 2 Gew.-% , bevorzugt von 0,001 bis 0,1 Gew.-%, bezogen auf das Gesamtgewicht der Fasern bzw. Textilien, auf die Fasern bzw. Textilien aufgebracht

Bevorzugt enthält die Zusammensetzung ZS ein Lösungsmittel L ausgewählt aus Wasser, polaren organischen Lösungsmitteln, unpolaren organischen Lösungsmitteln und Mischungen hiervon. Als organische Lösungsmittel L können insbesondere bekannte Lösungsmittel eingesetzt werden, die nicht leicht- oder hochentzündlich, nicht toxisch und mit Wasser im notwendigen Konzentrationsbereich mischbar sind. Beispielsweise kann das Lösungsmittel L enthalten (oder bestehen aus) Wasser, ein- oder mehrwertigen $C_{1-6}$-Alkoholen, Glykol, Glykolderivate, Polyglykole, Polyglykolether, Ether und Mischungen hiervon.

In einer bevorzugten Ausführungsform enthält die Zusammensetzung ZS mindestens ein Lösungsmittel L ausgewählt aus der Gruppe bestehend aus Wasser, Methyltriglycol, Ethylenglycol, Polyethylenglycol, Propylenglycol, Polypropylenglycol, Propylenglycol-monomethylether, Propylenglycol-dimethylether, Propylenglycol-monoethylether, Propylenglycol-diethylether, Methanol, Ethanol, Propanol und Butanol. Insbesondere enthält (oder besteht) das Lösungsmittel aus Methylentriglycol (Triethylenglycolmonomethylether) oder einer Mischung von Methylentriglycol und Wasser.

Die Zusammensetzung ZS enthält das mindestens eine Lösungsmittel L bevorzugt in einer Menge von 0 bis 99, 99999 Gew.-%, bevorzugt in einer Menge von 90 bis 99,99 Gew.-%, insbesondere in einer Menge im Bereich von 95 bis 99,9 Gew.-%, bezogen auf die Zusammensetzung ZS.

Bevorzugt enthält die Zusammensetzung ZS die folgenden Komponenten (oder besteht aus diesen):

0,0001 bis 100 Gew.-%, bevorzugt 0,0001 bis 10 Gew.-%, bevorzugt 0,001 bis 5 Gew.-%, besonders bevorzugt 0,01 bis 2 Gew.-%, insbesondere bevorzugt 0,01 bis 1 Gew.-%,
des mindestens einen hydrophilen Silan-Derivats S;
0 bis 99,9999 Gew.-%, bevorzugt 90 bis 99,9999 Gew.-%, bevorzugt 95 bis 99,999 Gew.-%, besonders bevorzugt 98 bis 99,99 Gew.-%, insbesondere bevorzugt 99 bis 99,99 Gew.-%, des mindestens einen Lösungsmittels L.

Optional kann die oben beschriebene Zusammensetzung ZS 0 bis 10 Gew.-%, bevorzugt 0 bis 1 Gew.-%, besonders bevorzugt 0,0001 bis 0,1 Gew.-%, bezogen auf die gesamte Zusammensetzung ZS, mindestens ein weiteres Additiv A enthalten. Typischerweise kann das mindestens eine weitere Additive A ausgewählt werden aus bekannten Textil-Hilfsmitteln. Beispielsweise kann es sich bei dem mindestens einen Additiv A um pH-Puffer,wie Natriumacetat oder eine Säure, z.B. Ameisensäure oder Essigsäure, Weichmacher, Hydrophobierungsmittel, Oleophobierungsmittel, Binder, Vernetzer, Flammschutzmittel, Textilfarbstoffe, Nähbarkeitsverbesserer und/oder Schmutzabweisende Mittel handeln.

Es ist auch die Verwendung eines antimikrobiellen Wirkstoffs als weiteres Additiv A möglich, welcher typischerweise bei der antimikrobiellen Ausrüstung von Fasern und Textilien eingesetzt wird, z.B. antimikrobiell wirkende Verbindungen mit quaternären Ammoniumfunktionen (z.B. Dimethyltetradecyl-3(trimethoxysilyl)propyl-ammonium chlorid oder Dimethyloctadecyl-3(trimethoxysilyl)propyl-ammonium chlorid); Silbersalze und -derivate, Thiohydroxamsäure-Derivat (z.B. Pyrithione, wie Natrium-Pyrithion oder Zink-Pyrithion), Isothiazolinon-Derivate (z.B. 1,2-Benzisothiazolin-3-on (BIT), Octylisothiazolin-3-on (OIT) und Methylisothiazolin-3-on (MIT), 4,5-Dichlor-2-n-octyl-4-isothiazolin-3-on (DCOIT), Butyl-1,2-Benzisothiazolin-3-on (BBIT)) oder Benzimidazol-Derivate (z.B. 2-(4-Thiazolyl)-1H-benzimidazol). In einer bevorzugten Ausführungsform enthält die Zusammensetzung ZS keinen antimikrobiellen Wirkstoff.

Insbesondere enthält die Zusammensetzung ZS die folgenden Komponenten (oder besteht aus diesen):

0,0001 bis 10 Gew.-%, bevorzugt 0,001 bis 5 Gew.-%, besonders bevorzugt 0,01 bis 2 Gew.-%, insbesondere bevorzugt 0,01 bis 1 Gew.-%,
des mindestens einen hydrophilen Silan-Derivats S wie oben beschrieben;
90 bis 99,9999 Gew.-%, bevorzugt 95 bis 99,999 Gew.-%, besonders bevorzugt 98 bis 99,99 Gew.-%, insbesondere bevorzugt 99 bis 99,99 Gew.-%, des mindestens einen Lösungsmittels L wie oben beschrieben, bevorzugt ausgewählt aus der Gruppe bestehend aus Wasser, Methyltriglycol, Ethylenglycol, Polyethylenglycol, Propylenglycol, Polypropylenglycol, Propylenglycol-monomethylether, Propylenglycol-dimethylether, Propylenglycol-monoethylether, Propylenglycol-diethylether, Methanol, Ethanol, Propanol und Butanol, besonders bevorzugt Methylentriglykol;
0 bis 10 Gew.-%, bevorzugt 0 bis 1 Gew.-%, besonders bevorzugt 0,0001 bis 0,1 Gew.-%, des mindestens einen weiteren Additives A.

Soweit nichts anderes angegeben ist, beziehen sich die Angaben in Gew.-% jeweils auf die Gesamtmenge der Zusammensetzung ZS. Die Angabe ppm meint im Sinne der vorliegenden Erfindung mg/kg.

Das Aufbringen der Zusammensetzung ZS auf die Fasern und/oder Textilien kann mit Hilfe eines bekannten

Verfahrens zur Ausrüstung von Fasern oder Textilien erfolgen. Bevorzugt erfolgt das Aufbringen der Zusammensetzung ZS in Schritt b mittels Foulard-Verfahren; Schaumauftrag; Sprühverfahren, insbesondere Sprayapplikation; Beschichtung, insbesondere Rakelverfahren, oder Auszugsmethode, bevorzugt mittels Foulard-Verfahren oder Auszugsmethode.

Es ist zudem möglich, die erfindungsgemäße Zusammensetzung ZS im Zusammenhang mit einem Wasch- und/oder Reinigungsschritt, z.B. im Rahmen einer üblichen Haushaltswäsche in einer Waschmaschine, auf die Fasern und/oder Textilien aufzubringen.

Das erfindungsgemäße Verfahren kann optional als Schritt c) das Waschen und/oder Trocknen der Fasern und/oder Textilien umfassen. Dies kann mittels der gängigen, dem Fachmann bekannten Verfahren, z.B. direkt nach dem Ausrüsten mit dem hydrophilen Silan-Derivat S, erfolgen.

Mit Hilfe des erfindungsgemäßen Verfahrens kann eine Ausrüstung von Fasern und/oder Textilien nahezu jeder Art erfolgen, beispielsweise von Fasern und/oder Textilien, die synthetische und/oder natürliche Fasern enthalten, wie Baumwolle; Regererat-Cellulose, z.B. Viskose, Lyocell (z.B. Tencel®); Hanf; Flachs; Leinen; Seide, Wolle, Polyester, Polyamid und Polyethylen Bevorzugt handelt es sich um Fasern und/oder Textilien, die im Wesentlichen aus Baumwolle, Regererat-Cellulose, Seide, Wolle, Polyester und/oder Polyamid bestehen. Gute Ergebnisse wurden auch mit Fasern und/oder Textilien erzielt, die synthetische Materialen enthalten oder die aus synthetischen Materialien bestehen. Als Beispiele seien Fasern und/oder Textilien aus Polyamid und/oder Polyester genannt. Bei den mit Hilfe des erfindungsgemäßen Verfahrens ausgerüsteten Textilien kann es sich vorzugsweise um gewebte Waren, gewirkte Waren, Vliese oder Garne handeln, aber auch andere Textilien können ausgerüstet werden. Insbesondere kann es sich bei den Textilien um textile Produkte der Bekleidungsindustrie (z. B. Damenoberbekleidung, Herrenbekleidung, Kinderbekleidung, Sport- und Freizeitbekleidung, Berufsbekleidung, Socken, Strümpfe und Unterwäsche), Bettzeug (z.B. Bettbezug und Laken), Matratzenstoffen, Heimtextilien, Sitzbezüge, Polsterstoffe, Textilien für Schuhe, Duschvorhänge, Filter, Teppiche, Schutzartikel (z. B. Maske und Bandage) und ähnliches handeln.

In einer bevorzugten Ausführungsform betrifft die Erfindung ein oben beschriebenes Verfahren zur Ausrüstung von Fasern und/oder Textilien, wobei es sich um Fasern und/oder Textilien handelt, welche synthetische Fasern, insbesondere Fasern aus Polyester, und/oder Polyamid enthalten. In einer bevorzugten Ausführungsform betrifft die Erfindung ein oben beschriebenes Verfahren zur Ausrüstung von Fasern und/oder Textilien, wobei es sich um Fasern und/oder Textilien handelt, welche im Wesentlichen aus synthetischen Fasern, insbesondere Fasern aus Polyester und/oder Polyamid bestehen. Insbesondere bevorzugt handelt es sich bei der Fasern und/oder Textilien um Sport- und Freizeitbekleidung, welche im Wesentlichen aus synthetische Fasern, insbesondere Fasern aus Polyester und/oder Polyamid bestehen.

Die Erfindung betrifft auch die mit einem hydrophilen Silan-Derivat S ausgerüsteten Fasern und/oder Textilien, hergestellt nach dem erfindungsgemäßen Ausrüstungsverfahren. Betreffend die einzelnen Komponenten, wie insbesondere hydrophiles Silan-Derivat S, Fasern und/oder Textilien und weitere Additive, gelten die oben - im Zusammenhang mit dem erfindungsgemäßen Ausrüstungsverfahren - genannten bevorzugten Ausführungsformen.

Ein weiterer Gegenstand der Erfindung betrifft die Verwendung eines hydrophilen Silan-Derivates S der allgemeinen Formel (I) wie oben beschrieben zur Verminderung der Anlagerung von Mikroorganismen an ein festes Substrat. Betreffend die einzelnen Komponenten, wie insbesondere Silan-Derivat S, Fasern und/oder Textilien und weitere Additive, gelten die oben - im Zusammenhang mit dem erfindungsgemäßen Ausrüstungsverfahren - genannten bevorzugten Ausführungsformen.

Bevorzugt betrifft die Erfindung die Verwendung eines hydrophilen Silan-Derivates S der allgemeinen Formel (I) wie oben beschrieben zur Verminderung der Anlagerung von Mikroorganismen an ein festes Substrat, wobei es sich bei dem festen Substrat um ein Substrat ausgewählt aus Glas, Keramik, Kunststoff, Metall, Fasern und Textilien, insbesondere ausgewählt aus Glas, Fasern und Textilien, handeln.

In einer bevorzugten Ausführungsform handelt es sich bei dem festen Substrat um ein Substrat ausgewählt aus Glas, Keramik, Kunststoff und Metall, insbesondere um Glas und/oder Kunststoff, bevorzugt um Glas.

Bevorzugt betrifft die Erfindung die Verwendung eines hydrophilen Silan-Derivates S ausgewählt aus den oben beschriebenen Verbindungen S1, S2, S3, S6, S10 und S11 zur Verminderung der Anlagerung von Mikroorganismen an ein festes Substrat.

Bevorzugt betrifft die Erfindung die Verwendung eines hydrophilen Silan-Derivates S ausgewählt aus den oben beschriebenen Verbindungen S1, S2, S3, S6, S10 und S11, bevorzugt ausgewählt aus den Verbindung S1 und S2, zur Verminderung der Anlagerung von Mikroorganismen an ein festes Substrat ausgewählt aus Glas, Keramik, Kunststoff und Metall, bevorzugt Glas.

Bevorzugt betrifft die Erfindung die Verwendung eines hydrophilen Silan-Derivates S ausgewählt aus den oben beschriebenen Verbindungen S1 und S3, bevorzugt S3, zur Verminderung der Anlagerung von Mikroorganismen an ein festes Substrat ausgewählt aus Glas, Keramik, Kunststoff und Metall, bevorzugt Glas.

Insbesondere betrifft die Erfindung die Verwendung eines hydrophilen Silan-Derivates S der allgemeinen Formel (I), wie oben beschrieben, zur Verminderung der Anlagerung von Mikroorganismen an ein festes Substrat, wobei das feste Substrat mit einer Zusammensetzung enthaltend mindestens ein hydrophiles Silan-Derivate S der allgemeinen

Formel (I), wie oben beschrieben, beschichtet wird. Insbesondere wird dabei das feste Substrat mit einer Zusammensetzung enthaltend mindestens ein hydrophiles Silan-Derivates S und mindestens ein Lösungsmittel beschichtet. Als Lösungsmittel L können insbesondere die oben im Zusammenhang mit dem erfindungsgemäßen Ausrüstungsverfahren beschriebenen Lösungsmittel verwendet werden. Insbesondere kann zur Beschichtung eines festen Substrats ein organischer Mono-, Di- oder Polyalkohol und/oder Wasser verwendet werden, bevorzugt werden $C_{1-6}$-Monoalkohole und/oder Wasser verwendet, besonderes bevorzugt werden Wasser, Ethanol, Methanol oder Mischungen hiervon verwendet.

Insbesondere kann zur Beschichtung des festen Substrats eine Zusammensetzung verwendet werden, die 0,00001 bis 100 Gew.-%, bevorzugt 0,001 bis 10 Gew.-%, insbesondere bevorzugt 0,01 bis 5 Gew.-% des hydrophilen Silan- Derivates S enthält.

Insbesondere betrifft die Erfindung die Verwendung einer Zusammensetzung, welche ein hydrophiles Silan-Derivates S wie oben beschrieben und mindestens ein Lösungsmittel enthält, zur Verminderung der Anlagerung von Mikroorganismen an ein festes Substrat.

Insbesondere kann es sich bei den Mikroorganismen um Bakterien, Viren, Pilze, Hefen und Algen handeln. Bevorzugt handelt es sich um Gram-positive und Gram-negative Bakterien oder Hefen, die typischerweise auf der menschlichen Haut vorkommen, z. B. Mikroorganismen der Gattung *Pseudomonas, Escherichia, Staphylococcus und Candida.* Vorzugsweise handelt es sich bei den Mikroorganismen um Mikroorganismen ausgewählt aus Vertretern der folgenden Gattungen:

> *Alternaria,* wie *Alternaria alternata; Aspergillus,* wie *Aspergillus niger, Aspergillus regens; Aureobasidium,* wie *Aureobasidium pullulans; Chaetomium,* wie *Chaetomium globosum; Coniophora,* wie *Coniophora puteana; Cladosporium,* wie *Cladosporium cladosporoides; Candida,* wie *Candida albicans; Lentinus,* wie *Lentinus tigrinus; Penicillium,* wie *Penicillium funiculosum; Rhodotorula,* wie *Rhodotorula rubra; Sclerophoma,* wie *Sclerophoma pityophila; Trichoderma,* wie *Trichoderma viride; Ulocladium,* wie *Ulocladium atrum; Escherichia,* wie *Escherichia coli; Pseudomonas,* wie *Pseudomonas aeruginosa; Staphylococcus,* wie *Staphylococcus aureus* oder *Staphylococcus epidermidis.*

bevorzugt ausgewählt aus Vertretern der Gattungen *Candida, Escherichia, Pseudomonas* und *Staphylococcus,* besonders bevorzugt ausgewählt aus Vertretern der Gattungen *Pseudomonas* und *Staphylococcus.*

Insbesondere handelt es sich bei den Mikroorganismen um Bakterien oder Hefen ausgewählt aus der Gruppe bestehend aus *Pseudomonas aeruginosa (P. aeruginosa), Escherichia coli (E. coli), Staphylococcus aureus (S. aureus), Staphylococcus epidermidis (S. epidermidis)* und *Candida albicans (C. albicans),* insbesondere bevorzugt aus *Pseudomonas aeruginosa (P. aeruginosa)* und *Staphylococcus aureus (S. aureus).*

Weiterhin wird ein Verfahren zur quantitativen Bestimmung der Anlagerung (Adhäsion) von Mikroorganismen an ein festes Substrat ausgewählt aus Fasern und/oder Textilien umfassend die folgenden Schritte beschrieben:

a')Herstellen einer wässrigen Suspension enthaltend einen oder mehrere verschiedene Mikroorganismen;

b')In Kontakt bringen der wässrigen Suspension enthaltend einen oder mehrere verschiedene Mikroorganismen mit dem festen Substrat, bevorzugt bei einer Temperatur im Bereich von 10 bis 50 °C und über einen Zeitraum von 0,5 bis 10 h;

c')Trennen des festen Substrats F von der überstehenden Suspension und Waschen des Substrats;

d')Abtrennen der angelagerten Mikroorganismen von dem festen Substrat mittels Einwirkung von Scherung und/oder Ultraschall und/oder Enzymen;

e')Bestimmen der Menge der abgetrennten Mikroorganismen.

Das beschriebene Bestimmungsverfahren erlaubt es insbesondere, die Menge der angelagerten Mikroorganismen an Fasern und/oder Textilien zu bestimmen, wobei diese Anlagerung oftmals den ersten Schritt bei der Ausbildung eines unerwünschten Biofilms darstellt. Es ist insbesondere nicht notwendig, dass sich ein vollständiger Biofilm ausbildet und bestimmt wird. Insbesondere wird bei dem beschriebenen Bestimmungsverfahren in erster Linie der erste entscheidende Schritt der Biofilm-Bildung, nämlich die Adhäsion (Anlagerung) der Mikroorganismen an der Faser- und/oder Textiloberfläche bestimmt. Insbesondere zeichnet sich das beschriebene Bestimmungsverfahren dadurch aus, dass das Inkontaktbringen der Fasern und/oder Textilien mit den Mikroorganismen über einen relativ kurzen Zeitraum, bevorzugt 1 bis 10 h, und in einem Medium erfolgt, das das Wachstum der Mikroorganismen nicht fördert, bzw. nicht ermöglicht (z.B. durch fehlende Nährstoffe).

Es hat sich überraschenderweise gezeigt, dass mit Hilfe des beschriebenen Bestimmungsverfahrens sicher, zuverlässig und schnell die Neigung von Fasern und/oder Textilien bestimmt werden kann, Bakterien anzulagern. Das beschriebene Bestimmungsverfahren eignet sich insbesondere dafür, die Qualität und Wirksamkeit einer anti-adhäsiven Beschichtung an Fasern und/oder Textilien zu bestimmen. Insbesondere kann das beschriebene Verfahren in einer oder mehreren Mikrotiterplatten durchgeführt werden und eignet sich dazu, in Form eines Hochdurchsatz-Verfahrens, z.B. eines Hochdurchsatz-Screening-Verfahrens, durchgeführt zu werden.

Da die vollständige Ausbildung eines Biofilms bei dem beschriebenen

Bestimmungsverfahren nicht notwendig ist, entfällt das aufwendige Waschen des Biofilms, wobei typischerweise unterschiedliche Mengen der Schleimschicht abgewaschen werden, was z.B. bei der Anfärbung mit Fluoreszenzfarbstoff und der anschließenden Fluoreszenzmessung zu Fehlern führen kann. Weiterhin vorteilhaft ist, dass auf den Einsatz von Nährlösung verzichtet werden kann, wodurch die nachfolgende Fluoreszenzbestimmung verlässlicher ist.

Insbesondere zeichnet sich das beschriebene Bestimmungsverfahren dadurch aus, dass es ein einfaches und zuverlässiges Verfahren darstellt, um beispielsweise die Wirksamkeit einer anti-adhäsiven Beschichtung auf Fasern und/oder Textilien zu bestimmen.

Das beschriebene Verfahren zur quantitativen Bestimmung der Bakterienadhäsion umfasst das Herstellen einer wässrigen Suspension enthaltend einen oder mehrere verschiedene Mikroorganismen (Schritt a').

Bevorzugt wird die wässrige Suspension enthaltend einen oder mehrere verschiedene Mikroorganismen durch Suspendieren einer definierten Menge einer Bakterienkultur, beispielsweise einer Übernachtkultur, in einem wässrigen Medium hergestellt. Bevorzugt ist das wässrige Medium der Suspension steril und enthält keine Nährstoffe (Nicht-Nährmedium).

Bevorzugt handelt es sich um eine Suspension enthaltend einen oder mehrere verschiedene Mikroorganismen und ein wässriges Medium. Insbesondere handelt es sich um ein wässriges Medium, das frei ist von Nährstoffen, insbesondere von Nährstoffen, die das Wachstum von Mikroorganismen fördern und/oder ermöglichen. Bevorzugt ist das wässrige Medium frei ist von Nähstoffen, die eine Kohlenstoff-Quelle darstellen, z.B. Kohlenhydrate, Proteine und Aminosäuren.

Bevorzugt handelt es sich bei dem wässrigen Medium um eine wässrige (insbesondere sterile) Lösung enthaltend mindestens ein Salz, insbesondere ausgewählt aus Natriumchlorid, Kaliumchlorid, Natrium-hydrogenphosphat, Dinatriumhydrogenphosphat, Kalium-dihydrogenphosphat und Dikalium-hydrogenphosphat. Bevorzugt handelt es sich bei dem wässrige Medium um eine isotonisch, wässrige Lösung enthaltend mindestens eines der oben genannten Salze. Insbesondere enthält das wässrige Medium mindestens eines der oben genannten Salze in einer solchen Konzentration, dass die Lösung einen osmotischen Druck aufweist, der dem osmotische Druck in den Zellen des menschlichen Körpers, z.B. des Blutplasma, entspricht (isotonische Lösung). Besonders bevorzugt handelt es sich bei dem wässrigen Medium um sterile, isotonische (physiologische) Kochsalzlösung (0,9 Gew.-% ige Kochsalzlösung), phosphat-gepufferte Salzlösung (PBS-Puffer) oder destilliertes/deionisiertes Wasser, insbesondere bevorzugt um isotonische Kochsalzlösung.

Bevorzugt handelt es sich um eine Suspension bestehend aus einem oder mehreren verschiedene Mikroorganismen und einem wässrigen Medium wie oben beschrieben. Bevorzugt handelt es sich um eine Suspension bestehend aus einem oder mehreren verschiedene Mikroorganismen und einem wässrigen Medium, wobei das wässrige Medium eine sterile, wässrige Lösungen mindestens eines der oben genannten Salze darstellt.

Bevorzugt enthält die wässrige Suspension mindestens einen der oben genannten Mikroorganismen, insbesondere ausgewählt aus Bakterien und Hefen. Bevorzugt enthalt die wässrige Suspension einen oder mehrere Mikroorganismen ausgewählt aus der Gruppe bestehend aus *Pseudomonas aeruginosa (P. aeruginosa), Escherichia coli (E. coli), Staphylococcus aureus (S. aureus), Staphylococcus epidermidis (S. epidermidis)* und *Candida albicans (C. albicans),* insbesondere bevorzugt aus *Pseudomonas aeruginosa (P. aeruginosa)* und *Staphylococcus aureus (S. aureus).* In einer bevorzugten Ausführungsform enthält die wässrige Suspension ausschließlich eine Art von Mikroorganismus, insbesondere ausschließlich einen der oben genannten Mikroorganismen.

Das Herstellen der wässrigen Suspension enthaltend einen oder mehrere verschiedene Mikroorganismen in Schritt a') erfolgt insbesondere dadurch, dass die Zellen einer Übernachtkultur des Mikroorganismus zentrifugiert, mit einem oben beschriebenen wässrigen Medium, bevorzugt mit isotonischer (physiologischer) Kochsalzlösung, gewaschen, und in einem oben beschriebenen wässrigen Medium, bevorzugt in isotonischer Kochsalzlösung, suspendiert werden.

Bevorzugt weist die wässrige Suspension in Schritt a') enthaltend einen oder mehrere verschiedene Mikroorganismen eine optische Dichte, typischerweise gemessen bei 595 nm, im Bereich von 0,8 bis 1,5; bevorzugt von 1 bis 1,5; insbesondere bevorzugt von 1 bis 1,3; beispielsweise etwa 1,2 auf. Typischerweise wird die optische Dichte der wässrigen Suspension in Schritt a') durch Zentrifugieren einer Mikroorganismen-Übernachtkultur, Aufnahme des Mikroorganismus in einem wässrigen Medium und einen oder mehrere Verdünnungsschritte auf den gewünschten Wert eingestellt.

Das beschriebene Verfahren zur quantitativen Bestimmung der Bakterienadhäsion umfasst das Inkontaktbringen der wässrigen Suspension enthaltend einen oder mehrere verschiedene Mikroorganismen mit dem festen Substrat

(Schritt b'). Bevorzugt erfolgt das Inkontaktbringen in Schritt b') bei einer Temperatur im Bereich von 10 bis 50 °C, bevorzugt von 20 bis 40 °C, insbesondere bevorzugt von 30 bis 35 °C. Bevorzugt erfolgt das Inkontaktbringen in Schritt b') über einen Zeitraum von 0,5 bis 10 h; bevorzugt 1 bis 5 h; insbesondere bevorzugt von 1 bis 3 h.

Bevorzugt umfasst Schritt b') das Inkontaktbringen der wässrigen Suspension enthaltend mindestens einen (bevorzugt genau einen) Mikroorganismus ausgewählt aus *Pseudomonas aeruginosa (P. aeruginosa), Escherichia coli (E. coli), Staphylococcus aureus (S. aureus), Staphylococcus epidermidis (S. epidermidis)* und *Candida albicans (C. albicans)* in isotonischer (physiologischer) Kochsalzlösung, wobei die optische Dichte der wässrigen Suspension einen Wert im Bereich von 0,8 bis 1,5 aufweist, mit dem festen Substrat, bevorzugt bei einer Temperatur im Bereich von 10 bis 50 °C, bevorzugt von 20 bis 40 °C, insbesondere bevorzugt bei 30 bis 35 °C, über einen Zeitraum von 0,5 bis 10 h; bevorzugt 1 bis 5 h; insbesondere bevorzugt von 1 bis 3 h.

Bevorzugt erfolgt das Inkontaktbringen in Schritt b') in einer Mikrotiterplatte.

Optional können nach der Adhäsionsphase, beispielsweise nach Schritt b') und/oder c'), die planktonischen Mikroorganismen (d.h. die Mikroorganismen, die nicht angelagert wurden) in der wässrigen Suspension bestimmt werden. Dies kann insbesondere zur Kontrolle dienen, insbesondere um die Menge an Mikroorganismen zu kontrollieren oder quantitativ zu bestimmen, die zur Adhäsion an das Substrat zur Verfügung gestanden haben. Bevorzugt wird die Menge der planktonischen Zellen nach der Adhäsionsphase, insbesondere nach Schritt c'), mittels der Bestimmung der optischen Dichte der wässrigen Suspension durchgeführt. Typischerweise erfolgt die Messung der optischen Dichte bei einer Wellenlänge von etwa 595 nm. Die gemessenen optischen Dichten (OD) können mittels einer Kalibrierung mit der Anzahl der Kolonien-bildenden Einheiten (CFU) korreliert werden.

Das beschriebene Verfahren zur quantitativen Bestimmung der Bakterienadhäsion umfasst das Trennen des festen Substrats F von der überstehenden Suspension und Waschen des Substrats (Schritt c').

Insbesondere umfasst Schritt c') das Herausnehmen des festen Substrats aus der wässrigen Suspension und Waschen des Substrats mit einem sterilen wässrigen Medium, insbesondere mit einem oben im Zusammenhang mit Schritt a') beschriebenen wässrigen Medium. Das Waschen kann typischerweise 1 bis 10 mal, bevorzugt 1 bis 5 mal, bevorzugt 1 bis 3 mal wiederholt werden.

Das Waschen des Substrats in Schritt c') kann insbesondere in einer Mikrotiterplatte erfolgen.

Das beschriebene Verfahren zur quantitativen Bestimmung der Bakterienadhäsion umfasst das Abtrennen der angelagerten Mikroorganismen von dem festen Substrat mittels Einwirkung von Scherung und/oder Ultraschall und/oder Enzymen (Schritt d').

In einer bevorzugten Ausführungsform erfolgt das Abtrennen der angelagerten Mikroorganismen von dem festen Substrat in Schritt d' mittels Behandlung des Substrats im Vortex und/oder mit Ultraschall. Es ist möglich die genannten mechanischen Verfahren mit einer Enzymbehandlung zu kombinieren.

Insbesondere umfasst Schritt d') das Inkontaktbringen des gewaschene Substrats erhalten aus Schritt c') mit einem wässrigen Medium, bevorzugt mit einem oben im Zusammenhang mit Schritt a') beschriebenen wässrigen Medium, und das Abtrennen der angelagerten Mikroorganismen von dem festen Substrat mittels Einwirkung von Scherung und/oder Ultraschall und/oder Enzymen.

Bevorzugt erfolgt das Abtrennen der angelagerten Mikroorganismen in Schritt d') in einer Mikrotiterplatte. Bevorzugt wird das feste Substrat erhalten aus Schritt c') in einer Mikrotiterplatte mit einem sterilen, wässrigen Medium, bevorzugt mit isotonischer Kochsalzlösung versetzt; die Mikrotiterplatte wird verschlossen und mittels Einwirkung von Scherung und/oder Ultraschall und/oder Enzymen, bevorzugt durch Einwirkung von Scherung und/oder Ultraschall, behandelt. Insbesondere wird somit nach Schritt d') eine wässrige Suspension erhalten, welche die abgetrennten Mikroorganismen enthält.

Für das wässrige Medium gelten die unter Schritt a') genannten bevorzugten Ausführungsformen in analoger Weise.

Das beschriebene Verfahren zur quantitativen Bestimmung der Bakterienadhäsion umfasst das Bestimmen der Menge der abgetrennten Mikroorganismen (Schritt e').

Bevorzugt umfasst Schritt e') vor der Bestimmung der Menge der abgetrennten Mikroorganismen das Herausnehmen des festen Substrats nach Schritt d') aus der wässrigen Suspension, welche die abgetrennten Mikroorganismen enthält.

Insbesondere wird ein Verfahren zur quantitativen Bestimmung der Anlagerung von Mikroorganismen wie oben beschrieben offenbart, wobei das Bestimmen der Menge der abgetrennten Mikroorganismen in Schritt e') mittels Fluoreszenzmessung, Biolumineszenz-Messung, Protein Assay oder Optical Density Proliferation Assay erfolgt. Die Menge der abgetrennten Mikroorganismen kann insbesondere direkt in der gemessenen relativen Einheit (z.B. in relativen Fluoreszenz-Einheiten (RFU)) oder durch die Anzahl der koloniebildende Einheiten (CFU) angegeben werden. Die Anzahl der koloniebildende Einheiten (CFU) kann insbesondere durch eine Kalibrierung der Messmethode erhalten werden.

Insbesondere kann die Bestimmung der Menge der abgetrennten Mikroorganismen in Schritt e') dadurch erfolgen, dass die Mikroorganismen mit einem Fluoreszenz-Farbstoff, insbesondere mit Syto9, angefärbt werden und

anschließend eine Auswertung über eine Fluoreszenzmessung erfolgt. Bevorzugt erfolgt die Auswertung in einem Mikroplatten-Reader. Weiterhin ist es möglich die Menge an abgetrennten Mikroorganismen mittels Anfärben mit einem Biolumineszenz-Farbstoffes (z.B. BacTiterGlo) und Biolumineszenz-Messung, mittels Protein assay (z.B. BCA assay, Bradford assay) oder mittels opticaldensity-proliferation-Assay zu bestimmen. Bevorzugt erfolgt die Bestimmung der Menge der abgetrennten Mikroorganismen in Schritt e') in einer Mikrotiterplatte. Bevorzugt erfolgen die Schritte d') und e') in einer Mikrotiterplatte, wobei Schritt e') die folgenden Schritte umfasst: Herausnehmen des festen Substrats aus der wässrigen Suspension, welche die abgetrennten Mikroorganismen enthält, aus den Näpfchen der Mikrotiterplatte, Zugabe eines Fluoreszenzfarbstoffes, insbesondere Syto9, und das Auswerten mittels Fluoreszenzmessung, typischerweise in einem Mikroplatten-Reader.

Es wird ein Verfahren zur quantitativen Bestimmung der Anlagerung von Mikroorganismen an ein festes Substrat beschrieben, wobei es sich um ein beschichtetes Substrat handelt, und die Menge der abgetrennten Mikroorganismen bestimmt in Schritt e') in Beziehung gesetzt wird zu der Menge der abgetrennten Mikroorganismen betreffend das jeweilige unbeschichtete Substrat. Dabei kann insbesondere die anti-adhäsiven Wirkung der Beschichtung mit Hilfe der Reduktion der angelagerten Mikroorganismen im Verhältnis zu dem jeweiligen unbeschichteten Substrat bestimmt werden. Bevorzugt können die entsprechenden in Schritt e') gemessenen, relativen, quantitativen Einheiten (z.B. relative Fluoreszenzeinheiten) gemäß der folgenden Gleichung in Beziehung gesetzt werden:

$$\text{Reduktion der angelagerten Bakterien [\%]} = [(RFU_{ub} - RFU_{b}) / RFU_{ub}]*100$$

mit

$RFU_{ub}$ = Relative Fluoreszenzeinheiten der abgetrennten Bakterien des unbeschichteten Substrats
$RFU_{b}$ = Relative Fluoreszenzeinheiten der abgetrennten Bakterien des beschichteten Substrats

Bevorzugt wird ein Verfahren zur quantitativen Bestimmung der Anlagerung von Mikroorganismen an ein festes Substrat beschrieben, wobei mindestens einer der Schritte a') bis e') ganz oder teilweise in einer Mikrotiterplatte durchgeführt wird. Bevorzugt werden die Schritte b') und d'); besonders bevorzugt die Schritte b'), c'), d') und e') ganz oder teilweise in einer Mikrotiterplatte durchgeführt.

Bevorzugt erfolgt das Inkontaktbringen der wässrigen Suspension in Schritt b') und das Abtrennen der angelagerten Mikroorganismen in Schritt d') in einer Mikrotiter-Platte. Besonders wird ein Bestimmungsverfahren beschrieben, wobei das Inkontaktbringen in Schritt b'), das Abtrennen der angelagerten Mikroorganismen in Schritt d') und die Bestimmung der Menge der abgetrennten Mikroorganismen in Schritt e) ganz oder teilweise in einer Mikrotiterplatte durchgeführt werden. Besonders bevorzugt wird ein Bestimmungsverfahren beschrieben, wobei das Inkontaktbringen in Schritt b'), das Waschen des festen Substrats in Schritt c'), das Abtrennen der angelagerten Mikroorganismen in Schritt d') und die Bestimmung der Menge der abgetrennten Mikroorganismen in Schritt e') ganz oder teilweise in einer Mikrotiterplatte durchgeführt werden.

Typischerweise können alle bekannten Formate, insbesondere die gängigen standardisierten Formate, und Formen von Mikrotiterplatten verwendet werden. Beispielsweise können Mikrotiterplatten aus Polystyrol, Polyvinylchlorid oder Glas verwendet werden, die typischerweise eine Näpfchen-Form ausgewählt aus Flachboden (F-Boden), Flachboden mit abgerundeten Ecken (C-Boden), konisch zulaufender Boden (V-Boden) und U-förmige Vertiefung (U-Boden) aufweisen.

Insbesondere kann das beschriebene Verfahren zur quantitativen Bestimmung der Anlagerung von Mikroorganismen an ein festes Substrat als Hochdurchsatz-Verfahren (High-Throughput-Verfahren), insbesondere als Hochdurchsatz-Screening-Verfahren (High-Throughput-Screening-Verfahren) durchgeführt werden.

Bevorzugt wird das beschriebene Verfahren zur quantitativen Bestimmung der Anlagerung von Mikroorganismen an ein festes Substrat als Hochdurchsatz-Verfahren (High-Throughput-Verfahren), insbesondere als Hochdurchsatz-Screening-Verfahren (High-Throughput-Screening-Verfahren) durchgeführt.

Insbesondere umfasst das beschriebene Bestimmungsverfahren, vor Schritt a') oder b') das Waschen und/oder Sterilisieren des festen Substrats.

Außerdem gelten für das Bestimmungsverfahren die oben beschriebenen bevorzugten Ausführungsformen betreffend Mikroorganismen und Fasern und/oder Textilien.

Die vorliegende Erfindung betrifft zudem ein Verfahren zur Ausrüstung von Fasern und/oder Textilien wie oben beschrieben, wobei durch die Ausrüstung eine Verminderung der Anlagerung von Mikroorganismen an die Fasern und/oder Textilien erreicht wird, und das mit dem oben beschriebenen quantitativen Bestimmungsverfahren kombiniert wird, um die Qualität der Ausrüstung zu beurteilen.

Eine Ausrüstung von Fasern und/oder Textilien zur Verminderung der Anlagerung von Mikroorganismen an

die Fasern und/oder Textilien wird insbesondere dann als ausreichend angesehen, wenn die Anlagerung von Bakterien im Vergleich zu einer nicht ausgerüsteten Kontrolle um mindestens 50 % reduziert wird, bevorzugt um 60% bis 100 % reduziert wird.

In diesem Zusammenhang betrifft die vorliegende Erfindung ein Verfahren zur Ausrüstung von Fasern und/oder Textilien gemäß Anspruch 1 und wie oben beschrieben, umfassend die Schritte

a) Bereitstellen einer Zusammensetzung ZS enthaltend mindestens ein hydrophiles Silan-Derivat S der allgemeinen Formel (I) und optional mindestens ein Lösungsmittel L;

b) Aufbringen der Zusammensetzung ZS auf die Fasern und/oder Textilien;

c) optional Waschen und/oder Trocknen der Fasern und/oder Textilien.

wobei die Ausrüstung auf eine Verminderung der Anlagerung von Mikroorganismen an die Fasern und/oder Textilien gerichtet ist, und wobei das Verfahren die folgenden zusätzlichen Schritte zur quantitativen Bestimmung der Anlagerung der Mikroorganismen an die Fasern und/oder Textilien umfasst:

a’1) Herstellen einer wässrigen Suspension enthaltend einen oder mehrere verschiedene Mikroorganismen;

b’1) In Kontakt bringen der wässrigen Suspension enthaltend einen oder mehrere verschiedene Mikroorganismen mit den ausgerüsteten Fasern und/oder Textilien aus Schritt b) oder c) oder mit einem Teil der ausgerüsteten Fasern und/oder Textilien aus Schritt b) oder c);

c’1) Trennen der Fasern und/oder Textilien von der überstehenden Suspension und Waschen der Fasern und/oder Textilien;

d’1) Abtrennen der angelagerten Mikroorganismen von den Fasern und/oder Textilien mittels Einwirkung von Scherung und/oder Ultraschall und/oder Enzymen;

e’1) Bestimmen der Menge der abgetrennten Mikroorganismen.

Die besonderen Ausführungsformen im Zusammenhang mit dem Verfahren zur Ausrüstung und dem Verfahren zur quantitativen Bestimmung der Anlagerung der Mikroorganismen gelten wie oben beschrieben in entsprechender Weise.

Figur 1 zeigt die Ergebnisse zur Waschbeständigkeit der anti-adhäsiven Wirkung (Reduktion der angelagerten Bakterien in %) gegenüber *S. aureus* betreffend eine Silan-Derivat-Beschichtung auf Polyestertextil-Proben gemäß Beispiel 3.2.

Figur 2 zeigt die Ergebnisse zur Waschbeständigkeit der anti-adhäsiven Wirkung (Reduktion der angelagerten Bakterien in %) gegenüber *P. aeruginosa* betreffend eine Silan-Derivat-Beschichtung auf Polyestertextil-Proben gemäß Beispiel 3.2.

Die Abkürzungen in den Figuren 1 und 2 sind im Folgenden erläutert:

S2, S3 und S5 bezeichnen die entsprechenden hydrophilen Silan-Derivate S2, S3 und S5, bzw. mit den entsprechenden Silan-Derivaten beschichteten Polyestertextil-Proben gemäß Beispiel 3.2;

A, B, und C bezeichnen die entsprechenden Vorbehandlungen der Polyestertextil-Proben (A), (B) und (C) gemäß Beispiel 3.2, d.h. A bezeichnet eine ungewaschenen Textilprobe; B bezeichnet eine Textilprobe, die zweimal bei 30 °C nur mit Wasser ohne Zugabe eines Waschmittels gewaschen wurde; C bezeichnet eine Textilprobe, die einmal bei 30 °C nach der Waschnorm EN ISO 6330, Methode 8A, unter Zugabe eines Waschmittels ECE B gewaschen wurde.

Zudem bezeichnen die Zahlen 0,1; 0,01 und 0,001 die entsprechenden Beschichtungskonzentrationen von 0,1 Gew.-%; 0,01Gew.-% und 0,001Gew.-%, hydrophiles Silan-Derivat S bezogen auf das Gewicht des beschichteten textilen Materials.

Die Erfindung wird durch die Ansprüche und durch die nachfolgenden Beispiele näher erläutert:

Beispiel 1 Beschichtung von Polyestertextil-Proben

Es wurden jeweils eine 10 Gew.-%ige Lösung der unten genannten hydrophilen Silan-Derivate S1 bis S4 in Methyltriglykol (Triethylenglykol-monomethylether, 2-[2-(2-Methoxyethoxy) ethoxy]ethanol hergestellt. Das hydrophile Silan-Derivat S5 wurde in Wasser gelöst, so dass eine 10 Gew.-%ige Lösung erhalten wurde.

Getestete hydrophile Silan-Derivate S

S1 Methoxy(polyethylenoxy)propyltrimethoxysilan (9-12 EO-Einheiten); (Hersteller Gelest, SIM 6492_72)

S2 Bis [3-(triethoxysilylpropoxy)-2-hydroxypropoxy)]polyethylenoxid (5-10 EO-Einheiten); (Hersteller Gelest, SIB 1824.2)

S3 N-(Triethoxysilylpropyl)-O-polyethylenoxid-urethan, (4-6 EO-Einheiten); (Hersteller Gelest, SIT 8192.0)

S4 N-(Triethoxysilylpropyl)-gluconamide (50 % ige Lösung in Ethanol) (Hersteller Gelest, SIT 8189.0)

S5 N-(Trimethoxysilylpropyl)ethylendiamin-triacetylsäure, Tri-Natrimsalz (45 %ige Lösung in Wasser) (Hersteller Gelest, SIT 8402.0)

Die Strukturformeln der hydrophilen Silan-Derivate S1 bis S3 sind oben in den Formeln (S1) bis (S3) angegeben. Die Silan-Derivate S4 und S5 (Vergleichsbeispiele) weisen die folgenden Strukturformeln auf:

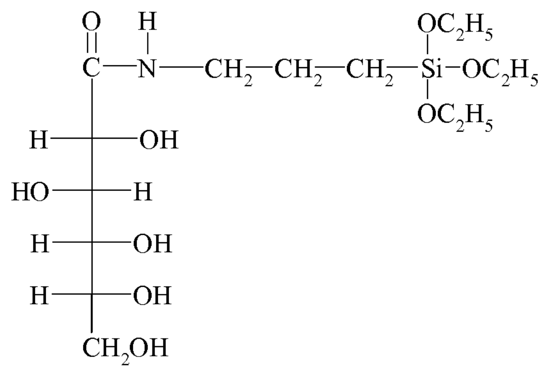

(S4)

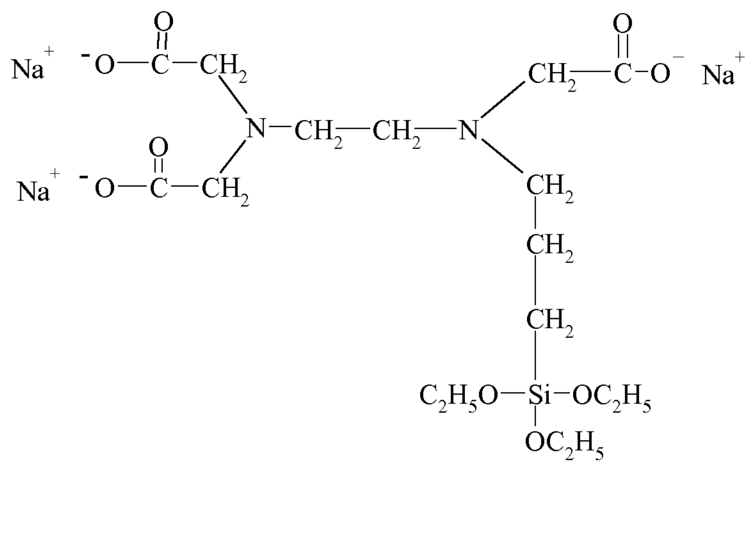

(S5)

Die oben beschriebenen Lösungen (Zusammensetzungen ZS) wurden mittels Foulard-Methode auf Polyestertextil-Proben aufgetragen, so dass sich Applikationskonzentrationen auf dem Textil von 0,1 Gew.-%, 0,01 Gew.-%, oder 0,001 Gew.-%, hydrophiles Silan-Derivat S bezogen auf Gesamtmenge des beschichteten Textils, ergaben.
In einer alternativen Verfahrensführung wurden direkt die oben genannten Handelsprodukte mittels Foulard-Methode auf Polyestertextil-Proben aufgetragen, so dass sich Applikationskonzentrationen auf dem Textil von 0,1 Gew.-%, 0,01 Gew.-%, oder 0,001 Gew.-%, hydrophiles Silan-Derivat S bezogen auf Gesamtmenge des beschichteten Textils, ergaben.

<u>Beispiel2: Verfahren zur quantitative Bestimmung der Anlagerung von Mikroorganismen an Textilien (Syto9-Assay)</u>

2.1 Herstellung der Textil-Titerplaten
Aus beschichteten und unbeschichteten Baumwoll- und Polyester-Textilien wurden runde Textilproben von einem Durchmesser von 0,6 cm ausgestanzt. Die Textilproben wurden auf einer Mikrotiterplatte fixiert. Die Textilproben wurden mit Aceton gewaschen und mit UV-Licht für 60 min sterilisiert.

2.2 Bakterienkultur und Bakterienvermehrung

Als Model-Mikroorganismen wurden verschiedenen gram-positive und gram-negative Bakterien und Hefen verwendet, insbesondere *Pseudomonas aeruginosa (P. aeruginosa), Escherichia coli (E. coli), Staphylococcus aureus (S. aureus), Staphylococcus epidermidis (S. epidermidis)* und *Candida albicans (C. albicans).*
Die Stämme wurden aus Glycerin-Bakterienkulturen frisch auf Trypton-Soja-Agar (Tryptic-Soy-Agar, TSA) ausgestrichen. Es erfolgte eine Inkubation über Nacht bei 33 °C. Als Nährmedium wurde eine Trypton-Soja-Bouillon (Tryptic-Soy-Broth, TSA) mit 30 % der vom Hersteller empfohlenen Menge angesetzt und mit 0,25 % Glucose ergänzt. Das Nährmedium wies einen pH-Wert von 7 bis 7,2 auf. Das Nährmedium wurde mit den Bakterienkulturen beimpft nach der Beimpfung bei 37 °C und 160 Upm über Nacht kultiviert.
Die so erhaltenen Übernacht-Kulturen wurden 15 min bei 8500 g zentrifugiert und mit 50 ml isotonischer Kochsalzlösung (0,9 Gew.-%) gewaschen. Es wurden Bakterien-Suspensionen in isotonischer Kochsalzlösungen hergestellt. Die optische Dichte der verschiedenen Bakterien-Suspensionen wurde bei 595 nm bestimmt und auf einen Wert von etwa 1,2 eingestellt.

2.3 Kalibrierung

Um die koloniebildende Einheit (colony forming units (CFU)) und die relativen Fluoreszence Einheiten (Relative Fluorescence Units (RFU)) in einer Standard-Kurve korrelieren zu können, wurden Verdünnungsserien der verschiedenen Mikroorganismen mit fünffacher Verdünnung hergestellt (80 µm isotonischer Kochsalzlösung und 20 µm Mikroorganismus-Lösung). Jeweils 5 µl Tropfen der verdünnten Lösungen wurden auf drei verschiedene Agarplatten (Nutrient agar, Plate Count Agar, and Tryptic-Soy-Agar) getropft. Die Agarplatten wurden umgedreht über Nacht bei 33 °C inkubiert. Die koloniebildende Einheit (CFU/ml) wurde am folgenden Tag durch Zählen bestimmt.
Im Falle einer Anfärbung mit Syto9 (Life Sciences) wurden die gleichen Verdünnungsserien zur gleichen Zeit in einer schwarzen Polystyrol-Platte hergestellt

2.4 Biofilm-Bildung

50 µl der finalen Mikroorganismus-Suspension in isotonischer Kochsalzlösung (0,9 Gew.-%) hergestellt wie unter 2.2 beschrieben wurden zu den beschichteten und unbeschichteten Textilproben gegeben, die auf den Mikrotiterplatten fixiert wurden (siehe 2.1). Die Mikrotiterplatten wurden verschlossen und mit Parafilm abgedichtet. Die Mikrotiterplatten wurden in einer Anti-Evaporation-Box platziert und bei 33°C für 2 h und bei 40 Upm inkubiert. Die erwartete koloniebildende Einheit (colony forming unit (CFU)) des Inokulums zu Start betrug $10^6$-$10^7$ CFU/ml.
Die planktonischen Zellen wurden nach der Bildung des Biofilm entfernt und dienten als Probe für die Bestimmung der optischen Dichte bei 595 nm. Dazu wurden die Proben bis zu einem Volumen von 150 µl aufgefüllt und 100 µl wurden für die optische Dichtebestimmung im Photometer entnommen. Die optische Dichte der entsprechenden Kontrolllösung mit steriler isotonischer Kochsalzlösung wurde von den bestimmten optischen Dichten abgezogen. Durch die Bestimmung der planktonischen Zellen nach der Biofilm-Bildung wurde die Menge der zur Adhäsion zur Verfügung stehenden Menge an Bakterien kontrolliert.
Die Textil-Proben und der entstandene Biofilm wurden dreimal mit 200 bis 300 µl isotonischer Kochsalzlösung gewaschen.

2.5 Mechanische oder enzymatische Entfernung der angelagerten Mikroorganismen

Die unter 2.4 erhaltenen Textilproben (nach Biofilm-Bildung) wurden mit 100 µl isotonischer Kochsalzlösung in Vertiefungen einer 96-Well-Mikrotiterplatte platziert.

Es wurden Untersuchungen zur Entfernung der angelagerten Mikroorganismen von den Textiloberflächen unter Verwirbelung (auf einem Vortex), Ultraschall, mit enzymatische Behandlung oder Kombinationen hiervon durchgeführt. Die Behandlung im Vortex betrug 5 min und wurde horizontal vorgenommen. Die Ultraschallbehandlung wurde im Ultraschallbad für 5 min durchgeführt. Für die enzymatische Entfernung wurde die Textilproben mit Enzymen, z.B. Trypsin oder eine Mischung von verschiedenen Enzymen, bei einer Inkubation von 1 h bei 37 °C behandelt.

Nach Abtrennung der angelagerten Bakterien wurden die Textilproben aus den Mikrotiterplatten entfernt.

Die Anzahl der abgetrennten Bakterien in der erhaltenen Suspension wurde mittels Anfärben mit Syto9 und Fluoreszenzmessung (Fluoreszenz-Filter mit Anregung bei 485/20 nm und Emission bei 528/20 nm) oder Anfärben mit BacTiterGlo und Biolumineszenz-Messung bestimmt. Weiterhin ist es möglich die Anzahl der angelagerten und abgetrennten Mikroorganismen mittels Protein assay (z.B. BCA Assay, Bradford Assay) oder Optical density proliferation Assay zu bestimmen.

Die relativen Messgrößen (z.B. Fluoreszenzeinheiten (RFU)) betreffend die beschichteten und unbeschichteten Textilien wurden verglichen und es konnte die Reduktion der angelagerten Bakterien in % gemäß der folgenden Formel bestimmt werden:

$$\text{Reduktion der angelagerten Bakterien [\%]} = [(RFU_{ub} - RFU_{b}) / RFU_{ub}]*100$$

mit

$RFU_{ub}$ = Relative Fluoreszenzeinheiten der abgetrennten Bakterien des unbeschichteten Substrats
$RFU_{b}$ = Relative Fluoreszenzeinheiten der abgetrennten Bakterien des beschichteten Substrats

Die statistische Signifikanz jeder Datenreihe wurde bestimmt. Die relativen Messeinheiten (z.B. relativen Fluoreszenzeinheiten (RFU)) können mit Hilfe von Kalibrationskurven (siehe 2.3) zu der koloniebildende Einheit pro ml (colony forming units (CFU/ml)), korreliert werden.

Beispiel 3: Untersuchung der ausgerüsteten Polyester-Textilproben

3.1 Bestimmungen der anti-adhäsiven Wirkung gegenüber verschiedenen Mikroorganismen

Polyestertextil-Proben wurden, wie unter Beispiel 1 beschrieben, mit jeweils einem hydrophilen Silan-Derivat S1 bis S5 ausgerüstet, so dass sich Applikationskonzentrationen auf dem Textil von 0,1 Gew.-% (hydrophiles Silan-Derivat S bezogen auf Gesamtmenge des beschichteten Textils) ergaben. Es wurden direkt die unter Beispiel 1 beschriebenen Handelsprodukte mittels Foulard-Methode auf Polyestertextil-Proben aufgetragen.

Die Bakterien-Adhäsion an den Polyestertextil-Proben wurde mittels des Mikrotiterplatten-Syto9-Assays, wie in Beispiel 2 beschrieben, untersucht.

Dazu wurden Polyester-Textilproben von einem Durchmesser von 0,6 cm ausgestanzt und in eine 96-Well-Mikrotiterplatte überführt. Die Bestimmung der Adhäsion von *S. aureus DSMZ* 20231 und *P. aeruginosa DSMZ* 1117 - als representative Vertreter von grampositiven und gram-negativen Bakterien - wurde wie in Beispiel 2 beschrieben durchgeführt, wobei die angelagerten Bakterien durch Behandlung im Vortex von den Textil-Proben abgetrennt wurden. Die quantitative Bestimmung der abgetrennten Bakterien erfolgte durch Anfärben mit Syto9 und Fluoreszenzmessung (Mikrotiterplatten-Syto9-Assay). Normale Kochsalz-Lösung wurde als sterile Kontrolle verwendet. Die Versuche wurden zweimal unabhängig voneinander wiederholt.

Die Ergebnisse zur Beschichtung auf PES-(Polyester)-Textilien sind in der folgenden Tabelle 1 dargestellt.

Tabelle 1: Ergebnisse zur Verminderung der Bakterienadhäsion an Polyestertextil-Proben

| | | Reduktion der angelagerten Bakterien [%] | |
|---|---|---|---|
| | | *S. aureus* | *P. aeruginosa* |
| Nr | Silan-Derivat S | | |
| 1 | S1 | 34 | 41 |

(fortgesetzt)

| | | Reduktion der angelagerten Bakterien [%] | |
|---|---|---|---|
| | | *S. aureus* | *P. aeruginosa* |
| Nr | Silan-Derivat S | | |
| 2 | S2 | 85 | 64 |
| 3 | S3 | 55 | 48 |
| 4 | S4 | 58 | 57 |
| 5 | S5 | 46 | 17 |

Die Reduktion der Adhäsion der beiden Mikroorganismen wurde gemäß der unter 2.5 angegebenen Formel berechnet. Es wurde gezeigt, dass sich Polyester-Textilien erfolgreich mit den beschriebenen hydrophilen Silan-Derivaten S1 bis S5 beschichten lassen und diese beschichteten Textilien gegenüber den unbeschichteten Textilien eine um bis zu 85 % reduzierte Adhäsion von Bakterien aufweisen.

3.2 Untersuchung der Waschbeständigkeit der anti-adhäsiven Beschichtung

Es wurden Polyestertextil-Proben mit den hydrophilen Silan-Derivaten S2, S3 und S5,wie in Beispiel 1 beschrieben, beschichtet, wobei jeweils eine 10 Gew.-% ige Lösung des Silan-Derivats, wie in Beispiel 1 beschrieben, mittels Foulard-Methode auf Polyestertextil-Proben aufgetragen wurde.

Es wurden jeweils Textilien mit Beschichtungskonzentrationen von 0,1 Gew.-%; 0,01 Gew.-% und 0,001 Gew.-%, hydrophiles Silan-Derivat bezogen auf das Gewicht des beschichteten textilen Materials, untersucht.

Die Textilproben wurden auf die Waschbeständigkeit der anti-adhäsiven Wirkung der Silan-Beschichtung untersucht. Die anti-adhäsive Wirkung wurde mittels des unter Beispiel 3 beschriebenen Syto9-Mikrotiterplatten-Assay in einer 96-Loch-Mikrotiterplatte bestimmt. Hierbei wurde jeweils getrennt die Adhäsion die beiden Stämme *S. aureus* und *P. aeruginosa* an den verschiedenen Textilproben betrachtet.

Es wurde die anti-adhäsive Wirkung jeweils von Textilproben mit folgenden Vorbehandlungen (A), (B) und (C) bestimmt:

(A) an einer ungewaschenen Textilprobe;

(B) an einer Textilprobe, die zweimal bei 30 °C nur mit Wasser ohne Zugabe eines Waschmittels gewaschen wurde;

(C) an einer Textilprobe, die einmal bei 30 °C nach der Waschnorm EN ISO 6330, Methode 8A, unter Zugabe eines Waschmittels ECE B gewaschen wurde.

Zu jeder Kombination wurden jeweils zwei unabhängige Experimente durchgeführt, um eine Aussage über die Verlässlichkeit der Ergebnisse zu erhalten.

Die Ergebnisse sind in der folgenden Tabellen 2 (Testung gegenüber *S. aureus*) und 3 (Testung gegenüber *P. aeruginosa*) zusammengefasst. Die Reduktion der Adhäsion der beiden Mikroorganismen ist jeweils in % bezogen auf die Adhäsion an unbeschichteten Textilproben angegeben.

Die Ergebnisse zur Waschbeständigkeit sind zudem in den Figuren 1 und 2 graphisch dargestellt. Figur 1 zeigt die Ergebnisse gemäß Tabelle 2 zur Waschbeständigkeit der anti-adhäsiven Wirkung (Reduktion der angelagerten Bakterien in %) gegenüber *S. aureus* betreffend eine Silan-Derivat-Beschichtung auf Polyestertextil-Proben. Figur 2 zeigt die Ergebnisse gemäß Tabelle 3 zur Waschbeständigkeit der anti-adhäsiven Wirkung (Reduktion der angelagerten Bakterien in %) gegenüber *P. aeruginosa* betreffend eine Silan-Derivat-Beschichtung auf Polyestertextil-Proben.

Es konnte gezeigt werden, dass die maximale Reduktion der Bakterien-Adhäsion von etwa 40 bis 60 % für alle untersuchten Proben bei den Waschbehandlungen erhalten bleibt. Es wurde gezeigt, dass die erfindungsgemäße anti-adhäsive Beschichtung eine hohe Waschbeständigkeit aufweist.

Tabelle 2: Waschbeständigkeit der anti-adhäsiven Wirkung gegenüber *S. aureus,* Polyestertextilproben, getestet unter Verwendung des Syto9-Assay

| Nr, | Silan-Derivat | Beschichtungskonzentration [%] | Waschvorbehandlung | Reduktion der angelagerten Bakterien [%] |
|---|---|---|---|---|
| 1 | S2 | 0,1 | (A) | 55 |
| 2 | | | (B) | 48 |
| 3 | | | (C) | 49 |
| 4 | | 0,01 | (A) | 71 |
| 5 | | | (B) | 31 |
| 6 | | | (C) | 66 |
| 7 | | 0,001 | (A) | 30 |
| 8 | | | (B) | 52 |
| 9 | | | (C) | - |
| 10 | S3 | 0,1 | (A) | 47 |
| 11 | | | (B) | 46 |
| 12 | | | (C) | 61 |
| 13 | | 0,01 | (A) | 63 |
| 14 | | | (B) | 34 |
| 15 | | | (C) | 58 |
| 16 | | 0,001 | (A) | 57 |
| 17 | | | (B) | 54 |
| 18 | | | (C) | 69 |
| 19 | S5 | 0,1 | (A) | 72 |
| 20 | | | (B) | 68 |
| 21 | | | (C) | 38 |
| 22 | | 0,01 | (A) | 44 |
| 23 | | | (B) | 22 |
| 24 | | | (C) | 53 |
| 25 | | 0,001 | (A) | 69 |
| 26 | | | (B) | 59 |
| 27 | | | (C) | 75 |

Tabelle 3: Waschbeständigkeit der anti-adhäsiven Wirkung gegenüber *P. aeruginosa,* Polyestertextilproben, getestet unter Verwendung des Syto9-Mikrotiterplatten-Assay

| Nr, | Silan-Derivat | Beschichtungskonzentration [%] | Waschvorbehandlung | Reduktion der angelagerten Bakterien [%] |
|---|---|---|---|---|
| 1 | S2 | 0,1 | (A) | 68 |
| 2 | | | (B) | 45 |
| 3 | | | (C) | 75 |
| 4 | | 0,01 | (A) | 79 |
| 5 | | | (B) | 36 |
| 6 | | | (C) | 75 |
| 7 | | 0,001 | (A) | 62 |
| 8 | | | (B) | 87 |
| 9 | | | (C) | 55 |
| 10 | S3 | 0,1 | (A) | 85 |
| 11 | | | (B) | 36 |
| 12 | | | (C) | 76 |
| 13 | | 0,01 | (A) | 65 |
| 14 | | | (B) | 37 |
| 15 | | | (C) | 71 |
| 16 | | 0,001 | (A) | 63 |
| 17 | | | (B) | 46 |
| 18 | | | (C) | 69 |
| 19 | S5 | 0,1 | (A) | 68 |
| 20 | | | (B) | 69 |
| 21 | | | (C) | 67 |
| 22 | | 0,01 | (A) | 78 |
| 23 | | | (B) | 54 |
| 24 | | | (C) | 73 |
| 25 | | 0,001 | (A) | 81 |
| 26 | | | (B) | 42 |
| 27 | | | (C) | 77 |

3.3 Untersuchung der antimikrobiellen Wirkung der beschichteten Textilien

Es wurde die antimikrobielle Aktivität der beschichteten Textilproben untersucht, hierbei wurden Polyestertextil-Proben, die jeweils mit einem Silan Derivat S1 bis S5 beschichtet wurden, mit Hilfe des ISO-standardisieren Verfahrens gemäß JIS L 1902 gegenüber *Staphylococcus aureus* bestimmt.

Hierbei wird in der Weise vorgegangen, dass die Textilprobe mit einer definierten Menge einer Keimsuspension (Inokulum) beimpft wurde. Es wurden Prüflinge mit einem Gewicht von etwa 0,4 g in einer dreifach Bestimmung untersucht.

Die Bebrütung der beimpften Textilprobe erfolgte in einem geschlossenen System bei 37 °C über einen Zeitraum von 18 Stunden. Nach der Bebrütung wurden die Bakterien einer definierten Menge einer Verdünnungslösung aufgenommen. Die Differenz der ausgezählten Bakterien der Textilprobe bei 0 Stunden im Vergleich zu der Textilprobe nach 18 Stunden diente als Auswertungsgrundlagen. Die antimikrobielle Wirksamkeit wird als logarithmischer oder prozentualer Wert angegeben. Hierbei entspricht eine Reduktion der Keimzahl von 0 % einer ungenügenden antimikrobiellen

Wirkung, eine Keimreduktion im Bereich von 0,1 bis kleiner 90 % einer ungenügenden antibakteriellen Wirkung und einer Keimreduktion von größer 90 % einer guten antibakteriellen Wirkung.

Die Ergebnisse betreffend die antimikrobielle Wirkung der beschichteten Textilien sind in der folgenden Tabelle 4 zusammengefasst.

Es wurde gezeigt, dass die beschichteten Polyester-Textilien so gut wie keine antimikrobielle Wirksamkeit gegenüber *Staphylococcus aureus* aufweisen, wobei Werte unter log 1 als "kein Effekt" gewertet werden.

Tabelle 4: Antimikrobielle Wirksamkeit auf Polyestertextil-Proben

| | | Antimikrobielle Wirksamkeit [log] | | |
|---|---|---|---|---|
| Silan-Derivat | Beschichtungskonzentration [%, bezogen auf Textil] | ungewaschen | Zweimal mit Wasser gewaschen | Einmal gewaschen bei 30 °C, ohne Waschmittel |
| unbehandelt | - | -0,5 | -0,1 | -0,5 |
| S2 | 0,1 | -0,2 | 0,00 | -0,4 |
| | 0,01 | -0,4 | 0,1 | -0,6 |
| | 0,001 | 0,2 | 0,1 | -0,1 |
| S3 | 0,1 | 0,0 | 0,3 | -0,4 |
| | 0,01 | -0,1 | 0,8 | -0,1 |
| | 0,001 | -0,5 | -0,3 | -0,8 |
| S5 | 0,1 | -0,5 | -0,4 | -0,6 |
| | 0,01 | -0,3 | -0,2 | -0,6 |
| | 0,001 | 0,5 | 0,9 | -0,3 |

Beispiel 4: Untersuchungen zur Anlagerung von Mikroorganismen an einer Glasoberfläche

4.1 Herstellung der beschichteten Glasproben

Die oben unter Beispiel 1 genannten Silan-Derivate S1, S3, S4 und S5 wurden verwendet um Beschichtungen auf Objektträgern aus Glas herzustellen. Als Kontrolle (Vergleichsprobe) K wurde ein unbeschichteter, mit Isopropanol gereinigter Objektträger aus Glas verwendet.

Zur Beschichtung der Objektträger wurden diese für 60 Sekunden in eine 0,1%-ige Lösung des entsprechenden Silan-Derivats in Ethanol/Wasser als Lösungsmittel mit einem Wasseranteil von 5 %. Die Lösungen wurden zuvor mit Essigsäure auf einen pH-Wert im bereich von 4 bis 5 eingestellt. Anschließend wurden die benetzten Objektträger 1 Stunde bei 160 °C getrocknet.

4.2 Bestimmung der Bakterienadhäsion über Fluoreszenzmikroskopie

Die mit der Bakteriensuspension benetzten Objektträger wurden mikroskopisch analysiert, um die anheftenden Bakterien zu bestimmen. Dazu wurde bereits der Testkultur ein Fluoreszenzfarbstoff zugesetzt, der die anheftenden Bakterien markiert und somit eindeutig identifizierbar an der Probenoberfläche markiert. Es wurde der Fluoreszenzfarbstoff DAPI (4',6-Diamidin-2-phenylindol) verwendet, welcher an DNS bindet und unter UV-Anregung fluoresziert. Zur Ermittlung des Anheftungsverhaltens wurden in 30 Messfenstern die Zellzahlen pro $cm^2$ bestimmt und das Anheftungsmuster bewertet. Die Auswertung des Cell Attachments erfolgte am Olympus IX 51 unter Einsatz des Bildverarbeitungsprogramms Labflow AnalySIS.

Als Bakteriensuspensionen wurden Suspensionen von Testkulturen der Stämme *Staphylococcus aureus* und *Escherichia coli* hergestellt und verwendet. Die Konzentration der Bakteriensuspension betrug $10^9$ Keime/ml.

Die Ergebnisse dieser Messungen sind in der folgenden Tabelle 5 zusammengestellt.

Tabelle 5: Ergebnisse der mikroskopischen Auswertung ermittelte Zellzahlen pro $cm^2$

| Silan-Derivat | Zellzahl [1/$cm^2$] | | Reduktion der Zellzahl gegenüber Kontrolle [%] | |
|---|---|---|---|---|
| | *S. aureus* | *E. coli* | *S. aureus* | *E. coli* |
| S1 | 110.000 | 296.000 | 76 | 63 |
| S3 | 77.000 | 233.000 | 83 | 71 |
| S4 | 383.000 | 389.000 | 17 | 51 |
| S5 | 219.000 | 306.000 | 52 | 61 |
| K (Kontrolle) | 460.000 | 791.000 | - | - |

Alle Proben zeigen im Vergleich zur Kontrolle Glas eine deutlich geringere Anheftung von Bakterien, wobei sich generelle als auch speziesbezogene Trends erkennen lassen.

Bei Anheftungsversuchen mit S. aureus ist festzustellen, dass eine weite Streuung der Ergebnisse vorliegt. So werden bei der Probe S4 nur 16% Anheftungsreduktion bezogen auf die Kontrolle ermittelt, während die Probe S3 eine Reduktion von 83% aufweist. Sehr gute Ergebnisse erzielten in diesem Versuch die Muster S1 und S3. Bei der mikroskopischen Begutachtung zeigten die beschichteten Oberflächen S4 wie die anderen Muster nur wenige traubenartige Aggregate von S. aureus, welche die typische Zellmorphologie der Staphylococcen darstellt. Die Kontrolle hingegen zeigte teilweise eine Zusammenlagerung der Zellen zu sehr großen Aggregaten, dies wurde auf den beschichteten Mustern nicht beobachtet.

Die Zellanhaftungen bei E.coli lagen für alle Proben vergleichsweise eng beieinander und zeigten eine mindestens 50-prozentige Zellzahlreduktion gegenüber der Kontrolle K (unbeschichteter Objektträger aus Glas). Die mikroskopischen Begutachtung zeigte, dass bei der Adhäsion von E. coli überwiegt eine Anheftung von Einzelzellen erfolgte, vereinzelt wurden fädige Aggregate beobachtet.

In beiden Testreihen konnten insbesondere die mit den Silan-Derivat S3 und S1 beschichteten Glasoberflächen sehr gute Ergebnisse erzielen.

## Patentansprüche

**1.** Verfahren zur Ausrüstung von Fasern und/oder Textilien umfassend die Schritte

a) Bereitstellen einer Zusammensetzung ZS enthaltend mindestens ein hydrophiles Silan-Derivat S der allgemeinen Formel (I)

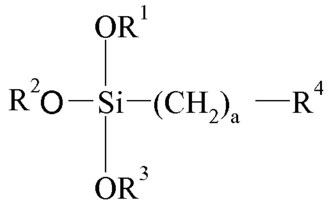

(I)

wobei

$R^1$, $R^2$ und $R^3$ unabhängig voneinander H oder $C_1$-$C_6$-Alkyl sind;
a eine ganze Zahl von 1 bis 10 ist;
$R^4$ ausgewählt ist aus:

i)

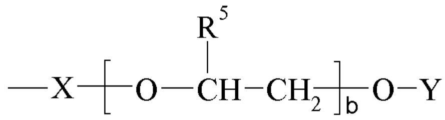

mit

X ist eine Bindung, -O-T-, -O-T-CH(OH)-, -O-T-CH(OH)-T'-, -O-C(=O)- oder -O-C(=O)-T-, wobei T und T' unabhängig voneinander ausgewählt sind aus $C_{1-12}$-Alkylen;
$R^5$ ist unabhängig voneinander H oder $C_{1-6}$-Alkyl;
Y ist H; $C_{1-12}$-Alkyl; $C_{1-12}$-Hydroxyalkyl, $C_{1-12}$-Haloalkyl, $C_{7-20}$-Arylalkyl, -C(=O)($C_{1-12}$-Alkyl); $-(CH_2)_a-Si(OR^1)(OR^2)(OR^3)$, $-T-O-(CH_2)_a-Si(OR^1)(OR^2)(OR^3)$; $-T-CH(OH)-O-(CH_2)_a-Si(OR^1)(OR^2)(OR^3)$, oder $-T-CH(OH)-T'-O-(CH_2)_a-Si(OR^1)(OR^2)(OR^3)$ mit $R^1$, $R^2$, $R^3$, T, T' und a wie oben definiert;
b ist eine Zahl von 0 bis 20;

oder (xv)

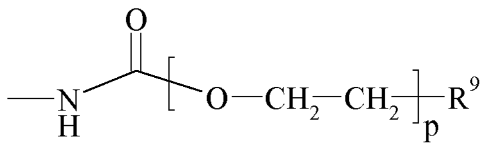

mit p = 1 bis 20 und $R^9$ ist ausgewählt aus -OH, $-OCH_3$, $-OCH_2CH_3$ und $-NH_2$;
und optional mindestens ein Lösungsmittel L;
b) Aufbringen der Zusammensetzung ZS auf die Fasern und/oder Textilien;
c) optional Waschen und/oder Trocknen der Fasern und/oder Textilien.

2. Verfahren gemäß Anspruch 1, **dadurch gekennzeichnet, dass** $R^1$, $R^2$ und $R^3$ unabhängig voneinander Methyl oder Ethyl sind und a = 3 ist.

3. Verfahren gemäß einem der Ansprüche 1 bis 2, **dadurch gekennzeichnet, dass** $R^4$ eine Gruppe ist ausgewählt aus:

(x)

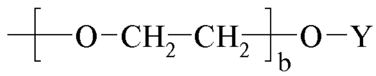

mit b = 2 bis 20 und Y ist ausgewählt aus H und $C_1$-$C_6$-Alkyl;
(xi)

-O-Y

mit Y ist ausgewählt aus H, $C_{1-6}$-Alkyl und -C(=O)($C_{1-12}$-Alkyl);
(xii)

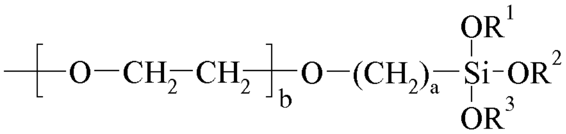

mit a = 2 bis 6, b = 2 bis 10 und $R^1$, $R^2$ und $R^3$ sind unabhängig voneinander Methyl oder Ethyl;
(xiii)

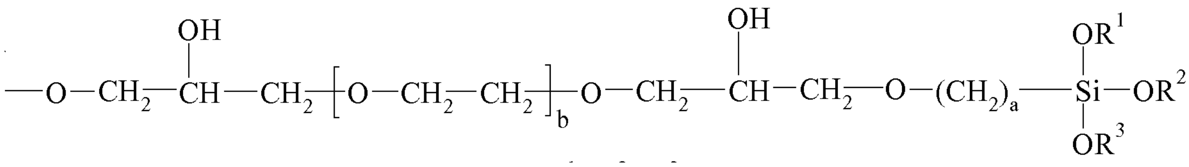

mit a = 2 bis 6, b = 2 bis 20 und $R^1$, $R^2$, $R^3$ sind unabhängig voneinander Methyl oder Ethyl;

4. Verfahren gemäß einem der Ansprüche 1 bis 3, **dadurch gekennzeichnet, dass** die Zusammensetzung ZS ein Lösungsmittel L ausgewählt aus der Gruppe bestehend aus Wasser, ein- und mehrwertigen $C_{1-6}$-Alkoholen, Glykol, Glykolderivate, Polyglykole, Polyglykolether, Ether und Mischungen hiervon, enthält.

5. Verfahren gemäß einem der Ansprüche 1 bis 4, **dadurch gekennzeichnet, dass** die Zusammensetzung ZS mindestens ein Lösungsmittel L ausgewählt aus der Gruppe bestehend aus Wasser, Methyltriglycol, Ethylenglycol, Polyethylenglycol, Propylenglycol, Polypropylenglycol, Propylenglycol-monomethylether, Propylenglycol-dimethylether, Propylenglycol-monoethylether, Propylenglycol-diethylether, Methanol, Ethanol, Propanol und Butanol enthält.

6. Verfahren gemäß einem der Ansprüche 1 bis 5, **dadurch gekennzeichnet, dass** die Zusammensetzung ZS 0,0001 bis 10 Gew.-%, bezogen auf die gesamte Zusammensetzung ZS, des mindestens einen hydrophilen Silan-Derivats S enthält.

7. Verfahren gemäß einem der Ansprüche 1 bis 6, **dadurch gekennzeichnet, dass** das Aufbringen der Zusammensetzung ZS in Schritt b mittels Foulard-Verfahren, Schaumauftrag, Sprühverfahren, Beschichtung oder Auszugsmethode erfolgt.

8. Verfahren gemäß einem der Ansprüche 1 bis 7, **dadurch gekennzeichnet, dass** es sich um Fasern und/oder Textilien handelt, welche synthetische Fasern enthalten.

9. Verfahren zur Ausrüstung von Fasern und/oder Textilien gemäß einem der Ansprüche 1 bis 8, wobei die Ausrüstung auf eine Verminderung der Anlagerung von Mikroorganismen an die Fasern und/oder Textilien gerichtet ist, und wobei das Verfahren die folgenden zusätzlichen Schritte zur quantitativen Bestimmung der Anlagerung der Mikroorganismen an die Fasern und/oder Textilien umfasst:

   a'1) Herstellen einer wässrigen Suspension enthaltend einen oder mehrere verschiedene Mikroorganismen;
   b'1) In Kontakt bringen der wässrigen Suspension enthaltend einen oder mehrere verschiedene Mikroorganismen mit den ausgerüsteten Fasern und/oder Textilien aus Schritt b) oder c) oder mit einem Teil der ausgerüsteten Fasern und/oder Textilien aus Schritt b) oder c);
   c'1) Trennen der Fasern und/oder Textilien von der überstehenden Suspension und Waschen der Fasern und/oder Textilien;
   d'1) Abtrennen der angelagerten Mikroorganismen von den Fasern und/oder Textilien mittels Einwirkung von Scherung und/oder Ultraschall und/oder Enzymen;
   e' 1) Bestimmen der Menge der abgetrennten Mikroorganismen.

10. Verwendung eines hydrophilen Silan-Derivates S der allgemeinen Formel (I) gemäß Anspruch 1 zur Verminderung der Anlagerung von Mikroorganismen an ein festes Substrat.

11. Verwendung nach Anspruch 10, **dadurch gekennzeichnet, dass** es sich bei dem festen Substrat um ein Substrat ausgewählt aus Glas, Keramik, Kunststoff, Metall, Fasern und Textilien handelt.

## Claims

1. A method for finishing fibers and/or fabrics, comprising the steps of

   a) providing a composition ZS comprising at least one hydrophilic silane derivative S of the general formula (I)

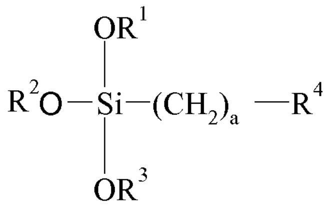

(I)

where

$R^1$, $R^2$ and $R^3$ independently of one another are H or $C_1$-$C_6$ alkyl;
a is an integer from 1 to 10;
$R^4$ is selected from:

i)

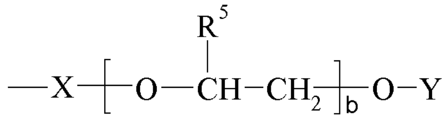

where

X is a bond, -O-T-, -O-T-CH(OH)-, -O-T-CH(OH)-T'-, -O-C(=O)- or -O-C(=O)-T-,
where T and T' independently of one another are selected from $C_{1-12}$ alkylene;
$R^5$ is independently of one another H or $C_{1-6}$ alkyl;
Y is H; $C_{1-12}$ alkyl; $C_{1-12}$ hydroxyalkyl, $C_{1-12}$ haloalkyl,
$C_{7-20}$ arylalkyl, -C(=O)($C_{1-12}$ alkyl); $-(CH_2)_a$-Si($OR^1$)($OR^2$)($OR^3$),
-T-O-$(CH_2)_a$-Si($OR^1$)($OR^2$)($OR^3$);
-T-CH(OH)-O-$(CH_2)_a$-Si($OR^1$)($OR^2$)($OR^3$), or
-T-CH(OH)-T'-O-$(CH_2)_a$-Si($OR^1$)($OR^2$)($OR^3$) with $R^1$, $R^2$, $R^3$, T, T' and a as defined above;
b is a number from 0 to 20;

or
(xv)

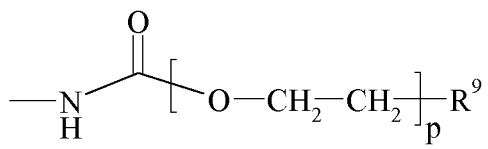

with p = 1 to 20 and $R^9$ is selected from -OH, $-OCH_3$, $-OCH_2CH_3$ and $-NH_2$; and optionally at least one solvent L;
b) applying the composition ZS to the fibers and/or fabrics;
c) optionally washing and/or drying the fibers and/or fabrics.

2. The method according to claim 1, **characterized in that** $R^1$, $R^2$ and $R^3$ independently of one another are methyl or ethyl and a=3.

3. The method according to any of claims 1 to 2, **characterized in that** $R^4$ is a group selected from:

(x)

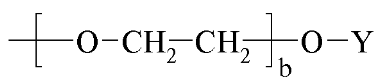

with b = 2 to 20 and Y is selected from H and $C_1$-$C_6$ alkyl;
(xi)

-O-Y

with Y is selected from H, $C_{1-6}$ alkyl and -C(=O)($C_{1-12}$ alkyl);
(xii)

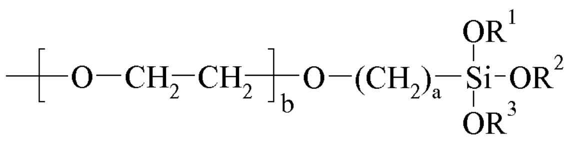

with a is 2 to 6, b is 2 to 10, and $R^1$, $R^2$ and $R^3$ are independently of one another methyl or ethyl; (xiii)

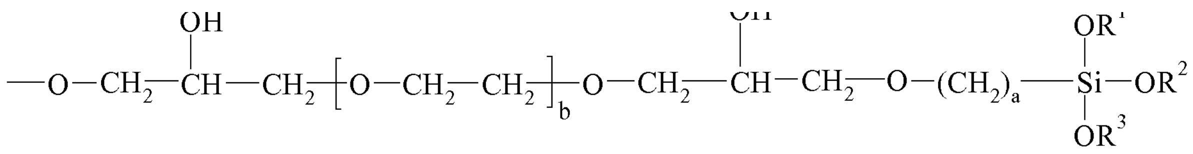

with a is 2 to 6, b is 2 to 20 and $R^1$, $R^2$ and $R^3$ are independently of one another methyl or ethyl.

4. The method according to any of claims 1 to 3, **characterized in that** the composition ZS comprises a solvent L selected from the group consisting of water, mono- and polyhydric $C_{1-6}$ alcohols, glycol, glycol derivatives, polyglycols, polyglycol ethers, ethers and mixtures thereof

5. The method according to any of claims 1 to 4, **characterized in that** the composition ZS comprises at least one solvent L selected from the group consisting of water, methyl triglycol, ethylene glycol, polyethylene glycol, propylene glycol, polypropylene glycol, propylene glycol monomethyl ether, propylene glycol dimethyl ether, propylene glycol monoethyl ether, propylene glycol diethyl ether, methanol, ethanol, propanol and butanol.

6. The method according to any of claims 1 to 5, **characterized in that** the composition ZS comprises 0.0001 to 10 wt%, based on the overall composition ZS, of the at least one hydrophilic silane derivative S.

7. The method according to any of claims 1 to 6, **characterized in that** the composition ZS is applied in step b by pad mangle methods, foam application, spraying methods, coating or exhaust method.

8. The method according to any of claims 1 to 7, **characterized in that** the fibers and/or fabrics comprise synthetic fibers.

9. The method for finishing fibers and/or fabrics according to any of claims 1 to 8, where the finishing is directed to reducing the attachment of microorganisms on the fibers and/or fabrics, and where the method comprises the following additional steps for quantitative determination of the attachment of the microorganisms on the fibers and/or fabrics:

   a'1) preparing an aqueous suspension comprising one or more different microorganisms;
   b'1) contacting the aqueous suspension comprising one or more different microorganisms with the finished fibers and/or fabrics from step b) or c) or with part of the finished fibers and/or fabrics from step b) or c);
   c'1) separating the fibers and/or fabrics from the supernatant suspension and washing the fibers and/or fabrics;
   d'1) removing the attached microorganisms from the fibers and/or fabrics by exposure to shearing and/or ultrasound and/or enzymes;
   e'1) determining the amount of microorganisms removed.

10. The use of a hydrophilic silane derivative S of the general formula (I) as in claim 1 for reducing the attachment of microorganisms on a solid substrate.

11. The use according to claim 10, **characterized in that** the solid substrate is a substrate selected from glass, ceramic,

plastic, metal, fibers and fabrics.

## Revendications

**1.** Procédé de traitement de fibres et/ou de textiles comprenant les étapes suivantes :

a) la préparation d'une composition ZS contenant au moins un dérivé de silane hydrophile S de formule générale (I)

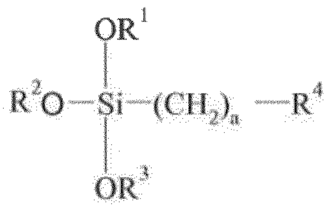

(I)

dans laquelle
$R^1$, $R^2$ et $R^3$ sont indépendamment les uns des autres H ou alkyle en $C_1$-$C_6$ ; a est un nombre entier de 1 à 10 ;
$R^4$ est choisi parmi : i)

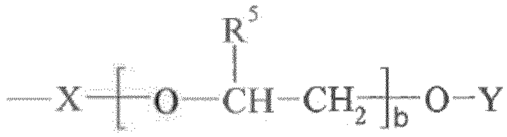

X étant une liaison, -O-T-, -O-T-CH(OH)-, -O-T-CH(OH)-T'-, -OC(=O)- ou -O-C(=O)-T-,
T et T' étant choisis indépendamment l'un de l'autre parmi alkylène en $C_{1-12}$ ;
les $R^5$ étant indépendamment les uns des autres H ou alkyle en $C_{1-6}$ ;
Y étant H, alkyle en $C_{1-12}$, hydroxyalkyle en $C_{1-12}$, haloalkyle en $C_{1-12}$, arylalkyle en $C_{7-20}$, -C(=O)(alkyle en $C_{1-12}$), $-(CH_2)_a-Si(OR^1)(OR^2)(OR^3)$, $-T-O-(CH_2)_a-Si(OR^1)(OR^2)(OR^3)$, $-T-CH(OH)-O-(CH_2)_a-Si(OR^1)(OR^2)(OR^3)$ ou $-T-CH(OH)-T'-O-(CH_2)_a-Si(OR^1)(OR^2)(OR^3)$ avec $R^1$, $R^2$, $R^3$, T, T' et a tels que définis précédemment ;
b étant un nombre de 0 à 20 ;
ou

(xv)

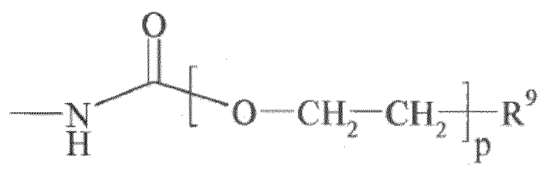

avec p = 1 à 20 et $R^9$ étant choisi parmi -OH, $-OCH_3$, $-OCH_2CH_3$ et $-NH_2$ ; et éventuellement au moins un solvant L ;
b) l'application de la composition ZS sur les fibres et/ou les textiles ;
c) éventuellement le lavage et/ou le séchage des fibres et/ou des textiles.

**2.** Procédé selon la revendication 1, **caractérisé en ce que** $R^1$, $R^2$ et $R^3$ sont indépendamment les uns des autres méthyle ou éthyle et a = 3.

**3.** Procédé selon l'une quelconque des revendications 1 à 2, **caractérisé en ce que** $R^4$ est un groupe choisi parmi :

(x)

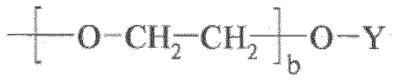

avec b = 2 à 20 et Y étant choisi parmi H et alkyle en $C_{1-6}$ ;

(xi)

-O-Y

Y étant choisi parmi H, alkyle en $C_{1-6}$ et -C(=O)(alkyle en $C_{1-12}$) ;

(xii)

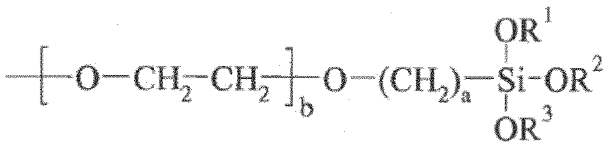

avec a = 2 à 6, b = 2 à 10 et $R^1$, $R^2$ et $R^3$ étant indépendamment les uns des autres méthyle ou éthyle ;

(xiii)

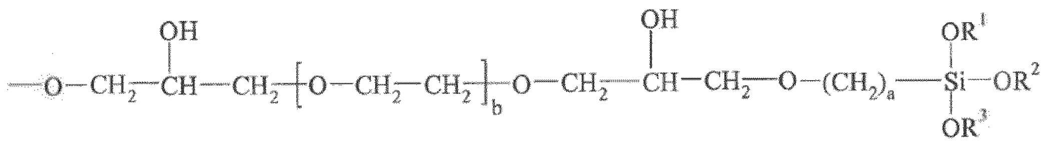

avec a = 2 à 6, b = 2 à 20 et $R^1$, $R^2$, $R^3$ étant indépendamment les uns des autres méthyle ou éthyle.

**4.** Procédé selon l'une quelconque des revendications 1 à 3, **caractérisé en ce que** la composition ZS contient un solvant L choisi dans le groupe constitué par l'eau, les alcools en $C_{1-6}$ mono- et polyvalents, le glycol, les dérivés de glycol, les polyglycols, les éthers de polyglycol, les éthers et leurs mélanges.

**5.** Procédé selon l'une quelconque des revendications 1 à 4, **caractérisé en ce que** la composition ZS contient au moins un solvant L choisi dans le groupe constitué par l'eau, le méthyltriglycol, l'éthylène glycol, le polyéthylène glycol, le propylène glycol, le polypropylène glycol, l'éther monométhylique de propylène glycol, l'éther diméthylique de propylène glycol, l'éther monoéthylique de propylène glycol, l'éther diéthylique de propylène glycol, le méthanol, l'éthanol, le propanol et le butanol.

**6.** Procédé selon l'une quelconque des revendications 1 à 5, **caractérisé en ce que** la composition ZS contient 0,0001 à 10 % en poids, par rapport à l'ensemble de la composition ZS, dudit au moins un dérivé de silane hydrophile S.

**7.** Procédé selon l'une quelconque des revendications 1 à 6, **caractérisé en ce que** l'application de la composition ZS à l'étape b a lieu au moyen de procédés de foulardage, d'application de mousse, de pulvérisation, de revêtement ou d'extraction.

**8.** Procédé selon l'une quelconque des revendications 1 à 7, **caractérisé en ce qu'**il s'agit de fibres et/ou de textiles qui contiennent des fibres synthétiques.

**9.** Procédé de traitement de fibres et/ou de textiles selon l'une quelconque des revendications 1 à 8, le traitement étant destiné à une réduction du dépôt de microorganismes sur les fibres et/ou les textiles, et le procédé comprenant les étapes supplémentaires suivantes pour la détermination quantitative du dépôt de microorganismes sur les fibres et/ou les textiles :

a'1) la préparation d'une suspension aqueuse contenant un ou plusieurs microorganismes différents ;

b'1) la mise en contact de la suspension aqueuse contenant un ou plusieurs microorganismes différents avec

les fibres et/ou textiles traités de l'étape b) ou c) ou avec une partie des fibres et/ou textiles traités de l'étape b) ou c) ;
c'1) la séparation des fibres et/ou textiles de la suspension surnageante et le lavage des fibres et/ou textiles ;
d'1) la séparation des microorganismes déposés des fibres et/ou textiles sous l'effet de cisaillements et/ou d'ultrasons et/ou d'enzymes ;
e' 1) la détermination de la quantité de microorganismes séparés.

**10.** Utilisation d'un dérivé de silane hydrophile S de formule générale (I) selon la revendication 1 pour la réduction du dépôt de microorganismes sur un substrat solide.

**11.** Utilisation selon la revendication 10, **caractérisée en ce que** le substrat solide est un substrat choisi parmi le verre, la céramique, le plastique, le métal, les fibres et les textiles.

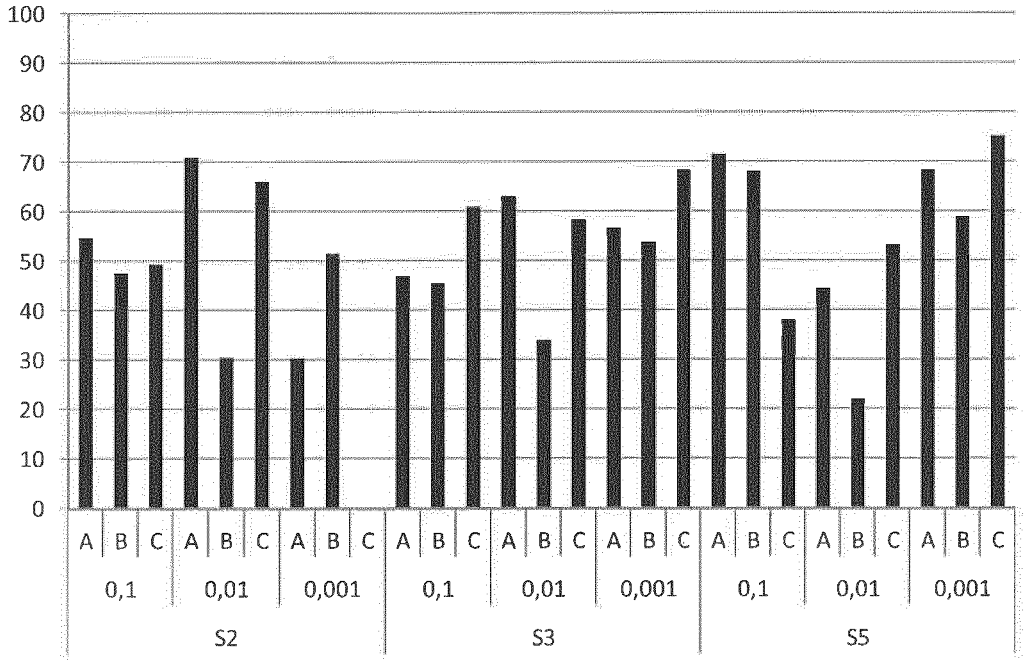
100
90
80
70
60
50
40
30
20
10
0
A B C A B C A B C A B C A B C A B C A B C A B C A B C
0,1 0,01 0,001 0,1 0,01 0,001 0,1 0,01 0,001
S2 S3 S5

Fig. 1

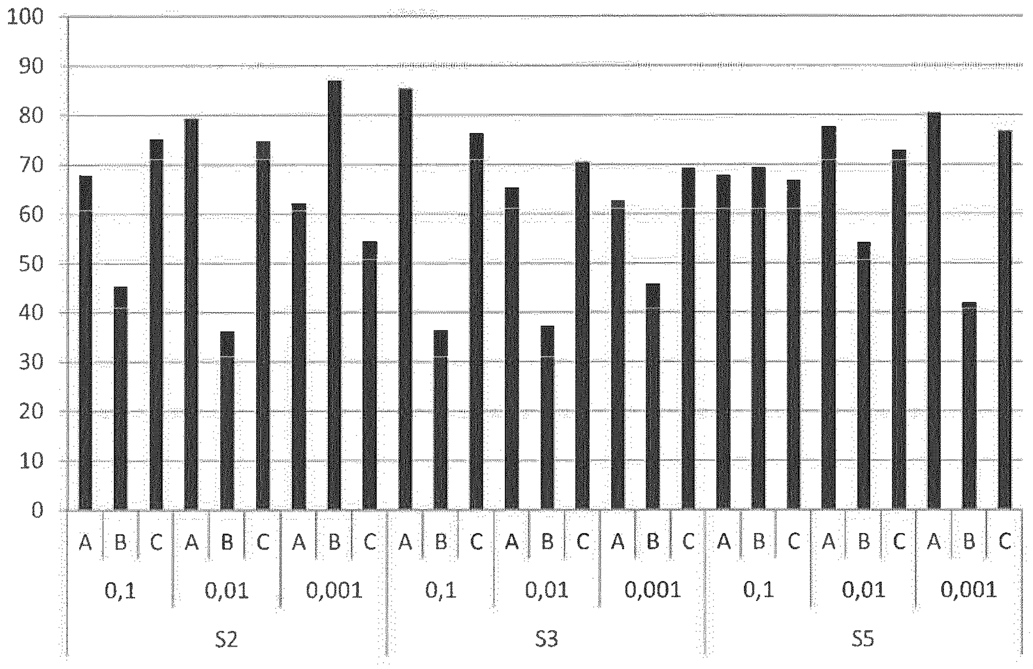
100
90
80
70
60
50
40
30
20
10
0
A B C A B C A B C A B C A B C A B C A B C A B C A B C
0,1 0,01 0,001 0,1 0,01 0,001 0,1 0,01 0,001
S2 S3 S5

Fig. 2

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2008089032 A
- WO 2003024897 A
- WO 2008049108 A
- US 20100112364 A
- US 3860709 B
- US 20100093666 A
- US 20050227092 A
- JP H0924089 B


### In der Beschreibung aufgeführte Nicht-Patentliteratur


- **M. CHARNLEY et al.** *Reactive & Functional Polymers,* 2011, vol. 71, 329-334
- **KINGSHOTT et al.** *Langmuir,* 2003, vol. 19, 6912-6921
- **TSIBOUKLIS et al.** *Contact Angle, Wettability and Adhesion,* 2006, vol. 4, 461-469
- **SRDJAN S. et al.** *APMIS,* 2007, vol. 115 (9), 891-899
- **PEETERS E. et al.** *Journal of Microbiological Methods,* 2008, vol. 72, 157-165
- **CIAG et al.** *Langmuir,* 2012, vol. 28, 1399-1407